# EUROPÄISCHE PATENTSCHRIFT

(11) **EP 1 948 042 B1**
(45) Veröffentlichungstag und Bekanntmachung des Hinweises auf die Patenterteilung: **17.10.2012**
(21) Anmeldenummer: 06818440.7
(22) Anmeldetag: 09.11.2006
(51) Int. Cl.: A61B 17/221, A46B 3/18

(54) **VORRICHTUNG ZUR ENTFERNUNG VON THROMBEN**
DEVICE FOR ELIMINATING THROMBOSES
DISPOSITIF POUR ELIMINER DES THROMBOSES

(30) Priorität: 09.11.2005 DE 102005053434
(43) Veröffentlichungstag der Anmeldung: 30.07.2008
(73) Patentinhaber: Phenox GmbH, 44801 Bochum (DE)
(72) Erfinder: MILOSLAVSKI, Elina, 70192 Stuttgart (DE); HANNES, Ralf, 44137 Dortmund (DE); PRACHT, Holger, 44628 Herne (DE); DIESTE, Fabian, 44807 Bochum (DE); MONSTADT, Hermann, 44797 Bochum (DE)
(74) Vertreter: Thiel, Christian
(86) Internationale Anmeldenummer: PCT/EP2006/010751
(87) Internationale Veröffentlichungsnummer: WO 2007/054307

(56) Entgegenhaltungen:
- BE-A- 333 551
- DE-A1- 3 921 071
- DE-B- 1 051 791

## Beschreibung

Die Erfindung betrifft eine Vorrichtung zur Entfernung von Fremdkörpern und Thromben aus Körperhohlräumen und Blutgefäßen mit einem Führungsdraht, der ein oder mehrere distale Elemente aufweist.

Thromboembolische Erkrankungen wie Herzinfarkt, Lungenembolie, periphere Thrombose, Organembolien, etc. werden typischerweise durch einen Thromboembolus (im Folgenden kurz Thrombus), also einen viskoelastischen Blutklumpen aus Blutplättchen, Fibrinogen, Gerinnungsfaktoren etc., ausgelöst, der sich in einem Blutgefäß festgesetzt hat und dieses ganz oder teilweise verschließt. Der Verschluss von Organarterien führt dabei zu einer Unterbrechung der Versorgung des abhängigen Gewebes mit Sauerstoff und Nährstoffen. Der Störung des Funktionsstoffwechsels mit Funktionsvertust folgt innerhalb kurzer Zeit das Erliegen des Strukturstoffwechsels mit dem Untergang des betroffenen Gewebes (Infarkt). Die häufigsten hiervon beim Menschen betroffenen Organe sind das Herz und das Gehirn. Solche Veränderungen betreffen aber auch die Extremitätenarterien und die Lungenarterien. Venöse Thrombosen und thromboembolische Verschlüsse kommen gehäuft in den Bein- und Beckenvenen vor. Das Krankheitsbild des thrombotischen Verschlusses eines intrakraniellen Sinus kann durch die Störung der venösen Drainage des Hirngewebes zu schweren Hirnblutungen führen.

Angesichts der Schwere der durch Thromboembolien ausgelösten Krankheitsbilder und der Häufigkeit dieser Erkrankungen sind verschiedene Techniken zur Auflösung oder Entfernung von Thromben bekannt.

So ist es bekannt, solche Patienten mit thrombolytischen Mitteln wie Streptokinase oder Urokinase oder mit Antikoagulantien zu behandeln, was zur Thrombolyse oder zur Eindämmung des Thrombenwachstums führen soll. Da diese Behandlungsmethoden meist zeitintensiv sind, werden sie oftmals zusammen mit Eingriffen kombiniert, die der mechanischen Zerkleinerung oder Entfernung des Thrombus bzw. Embolus dienen.

Neben offenen chirurgischen Eingriffen kommen im Stand der Technik zunehmend transluminale bzw. endovaskuläre, Katheter geführte interventionelle Therapieformen zum Einsatz, da diese weniger invasiv sind. So ist es bekannt, den Thrombus mittels Unterdruck erzeugenden Saug-Kathetern oder mechanisch mit Kathetern, welche mit Fangkörben, Wendeln, Haken oder dergleichen versehen sind, aus dem Körper des Patienten zu entfernen, siehe US 6 245 089 B1, US 5 171 233 A1, Thomas E. Mayer et al., Stroke 2002 (9), 2232.

Der Nachteil der bekannten transluminalen Vorrichtungen besteht darin, dass auch diese den Thrombus häufig nicht vollständig entfernen können, und die Gefahr besteht, dass der Thrombus oder Fragmente davon sich freisetzen und im Blutstrom zu kleinlumigeren Gefäßen weiterziehen, welche schwerer zu erreichen und zu behandeln sind. Des weiteren eignen sich die im Stande der Technik bekannten Vorrichtungen aufgrund ihrer Dimensionen und/oder geringen Flexibilität nur ungenügend zur Entfernung von Thromben aus besonders kleinlumigen oder stark gewundenen Gefäßen, wie denen des Gehirns.

So ist aus der US 2002/0049452 eine Vorrichtung mit einem Katheter zur Entfernung von Thromben bekannt, an dessen distalem Ende Fangarme aus einem Formgedächtnismaterial angebracht sind, die im komprimierten Zustand am Katheter anliegen und sich in der expandierten Konfiguration radial vom Katheter nach außen erstrecken. Nach der durch die Körpertemperatur ausgelösten Einnahme der expandierten Konfiguration sollen sich die Fangarme im Thrombus verhaken und diesen bei Rückführung des Katheters in einen weiteren Katheter aus dem Blutgefäß mit sich ziehen. Der Nachteil dieser Vorrichtung besteht darin, dass sie entweder zur Kühlung der Fangarme unter die Umwandlungstemperatur, bis diese in den Blutstrom gelangen, in einem Sekundärkatheter, der diese Kühlung ermöglicht, an dem Thrombus vorbei manövriert werden muß; oder der mit den Fangarmen versehene Katheter muss in seinem Inneren eine Heizvorrichtung tragen, die die Erhitzung auf die Umwandlungstemperatur nach dem Erreichen des Thrombus ermöglicht. Diese konstruktiven Erfordernisse sind zum einen sehr aufwendig und somit auch störanfällig und machen allein aufgrund ihres physikalischen Umfanges die Behandlung besonders kleinlumiger Gefäße unmöglich.

DE 39 21 071 A1 offenbart eine Vorrichtung gemäß dem Oberbegriff des Anspruchs 1.

Angesichts der mit dem Stand der Technik verbundenen Nachteile besteht die Aufgabe der Erfindung somit in der Bereitstellung einer Vorrichtung zur Entfernung von Fremdkörpern und Thromben aus Körperhohlräumen und Blutgefäßen, welche das operative Risiko bei der Entfernung von Thromben vermindert und die Behandlung besonders kleinlumiger Gefäße erlaubt.

Die Aufgabe wird erfindungsgemäß gelöst durch eine Vorrichtung zur Entfernung von Fremdkörpern und Thromben aus Körperhohlräumen und Blutgefäßen mit einem Führungsdraht und ein oder mehreren distalen Elementen, die mindestens zwei miteinander verdrehte Seelendrähte umfassen, zwischen denen Fasern quer zum Verlauf der Seelendrähte eingelegt und mit den Seelendrähten verdrillt sind, so dass die Fasern vom distalen Element radial nach außen hervorstehen, wobei die radiale Ausdehnung der Fasern von proximal nach distal zunimmt.

Das distale Element hat somit die Form einer Bürste, von der borstenartige Fasern nach außen stehen. Die Fasern dienen dazu, einen Thrombus festzuhalten und zu stabilisieren, indem sie sich im Thrombus verhaken und auf diese Weise seine Rückführung erleichtern. Gleichzeitig sind die Fasern/Borsten flexibel, so dass sie sich beim Vorschieben der Vorrichtung aufgrund des mechanischen Widerstands in proximale Richtung an das distale Element anlegen. Bei Wegfall des äußeren Zwangs durch den Mikrokatheter entfaltet sich die Bürste zur vollen Bürstenstruktur. Entsprechend müssen die Fasern über eine hinreichende Steifigkeit verfügen, um ihren Zweck des Festhaltens des Thrombus zu erfüllen, gleichzeitig aber auch hinreichend flexibel und biegsam sein, um durch einen Katheter geführt werden zu können und die Gefäßwände nicht zu verletzen.

Die Fasern oder Borsten sind geeignet, einen Thrombus festzuhalten und zu stabilisieren, insbesondere dann, wenn sie aus thrombogenen Materialien bestehen oder damit ausgerüstet sind.

Die Fasern/Borsten können auch aus einem Naturstoff, Polymermaterial, Metall, keramischen Material, Glas oder Kombinationen davon bestehen. Besonders bevorzugt sind Polymermaterialien.

Als Materialien eignen sich hier vor allem Polyurethan, Polyacryl, Polyester, Polytetrafluorethylen, Polyamid oder Polyethylen, aufgrund seiner peptidähnlichen Bindungsstruktur vor allem Polyurethan und Polyamid, z. B. Nylon, was eine besonders gute Anbindung/Adhäsion des Thrombus an den Fasern ermöglicht.

Neben Polymermaterialien kommen vor allem metallische Materialien in Frage. Geeignete Metalle sind alle Metalle, die ohne Nachteil für die Patienten zur Behandlung herangezogen werden können. Insbesondere geeignet sind für den genannten Zweck Edelstahlfasern aus Metalllegierungen mit Formgedächtniseigenschaften, wie beispielsweise Nitinolfasern. Fasern aus Formgedächtnismaterialien haben den Vorteil, dass sie unter dem äußeren Zwang eines Mikrokatheters in einer ersten, eng anliegenden Form vorliegen können und nach Freisetzung aus dem Mikrokatheter in einer zweiten orthogonalen Form mit frei abstehenden Fasern. Weiterhin sind Gold und Platin geeignete Materialien. Ebenfalls geeignet sind keramische Materialien, Glasfasern und Kohlenstofffasern.

Zur Behandlung besonders kleinlumiger Gefäße eignen sich insbesondere Fasern einer Länge von 0,5 bis 6 mm und vorzugsweise 0,5 bis 3 mm, so dass auch bei radialer Anordnung der Fasern ein Außendurchmesser des die Fasern tragenden Teils des distalen Elements von 1 bis maximal 12 mm erreicht wird. Zur besonders atraumatischen Behandlung sollte dieser Außendurchmesser etwas kleiner dimensioniert sein, als der Innendurchmesser des betreffenden Blutgefäßes.

Die Fasern erstrecken sich zweckmäßigerweise über eine Länge des distalen Elementes, die 0,5 bis 5 cm ausmacht. Zur Gewährleistung einer ausreichend guten Verankerung des Thrombus ist es zweckmäßig, wenn die Fasern in einer Dichte von 20 bis 100 pro cm an dem distalen Element des Führungsdrahtes angeordnet sind.

Die erfindungsgemäß zum Einsatz kommenden Fasern oder Borsten stehen vorzugsweise in einem Winkel von 70° bis 110°, vorzugsweise in einem Winkel von 80° bis 90° von der Längsachse der Vorrichtung ab. Diese Winkelangaben sind so zu verstehen, dass Winkel < 90° eine proximale Ausrichtung der Fasern, Winkel > 90° eine distale Ausrichtung der Fasern bezeichnen. Ausführungsformen mit einem Winkel, der etwas kleiner ist als 90° sind besonders atraumatisch beim Vorschieben in das Gefäß bzw. durch den Thrombus und bewirken gleichzeitig eine besonders gute Verankerung im Thrombus beim Zurückziehen aus dem Blutgefäß.

Zweckmäßigerweise ist der Führungsdraht aus einem medizinischen Edelstahl oder einem Formgedächtnismaterial, vorzugsweise Nitinol, hergestellt. Dabei ist es zweckmäßig, wenn der Führungsdraht einen Außendurchmesser von 0,2 bis 0,4, vorzugsweise 0,22 bis 0,27 mm aufweist. Eine typische Länge eines Führungsdrahtes liegt zwischen 50 bis 180 cm, kann aber auch mehrere Meter betragen.

Gemäß einer besonders bevorzugten Ausführungsform der Vorrichtung sind die Fasern beschichtet. Dies kann beispielsweise eine neutrale Beschichtung aus Parylen oder Polytetrafluorethylen (Teflon) sein, aber auch eine solche mit Kollagen oder mit einem die Blutgerinnung fördernden Material, vorzugsweise einem oder mehreren Gerinnungsfaktoren. Diese Ausführungsform dient der verstärkten Verankerung der Fasern im Thrombus und vermindert die Gefahr, dass der Thrombus so weit zerfällt, dass Teile des Thrombus im Blutgefäß zurückbleiben oder sich im Blutstrom freisetzen können.

Es wurde überraschend gefunden, dass eine thrombogene Ausrüstung der Fasern/Borsten zu einer erheblichen Stabilisierung des Thrombus an der erfindungsgemäßen Vorrichtung führen. Es ist dabei dem Operateur überlassen, die erfindungsgemäße Vorrichtung so mit dem Thrombus in Kontakt zu bringen und zu belassen, dass über eine gewisse Einwirkungszeit der thrombogenen Elemente ein "Festwachsen" des Thrombus an der Vorrichtung gefördert wird. Dieses Festwachsen erfolgt in relativ kurzer Zeit an thrombogenen Fasern/Borsten, teilweise innerhalb einiger Minuten. Dies beugt nicht nur einem Zerfall des Thrombus vor, wie er mit vielen, kommerziell erhältlichen Vorrichtungen beobachtet wird, sondern erleichtert auch die Einholung des Thrombus in einen Katheter und seine Extraktion aus dem vaskulären System. Die dazu besonders geeigneten thrombogenen Materialien und Beschichtungen sind dem Fachmann aus der Literatur bekannt. Besonders eignen sich etwa einer oder mehrere der Faktoren Fibrin, Thrombin, Faktor XIII und/oder Faktor VIII.

Bei der Anwendung der Erfindung geht man folgendermaßen vor: Die Vorrichtung wird mit Hilfe eines kleinlumigen Mikrokatheters an den Einsatzort gebracht. Es ist gleichermaßen möglich, die Vorrichtung im Mikrokatheter 1) zuerst distal des Thrombus zu manövrieren und dann zurückzuziehen, 2) im Bereich des Thrombus aus dem Mikrokatheter freizusetzen, 3) proximal des Thrombus aus dem Mikrokatheter heraus zuschieben und dann anterograd den Thrombus zu penetrieren. Beim Vorschieben der Vorrichtung legen sich die biegsamen Fasern aufgrund des mechanischen Widerstandes in proximale Richtung an das distale Element an, beim Zurückziehen dagegen stellen sie sich auf, verhaken sich im Thrombus und unterstützen so die Rückführung in einen größeren als den ursprünglich verwendeten Mikrokatheter. Hierbei handelt es sich üblicherweise um einen Führungskatheter (guide catheter). Durch einen solchen Führungskatheter kann man einen Mikrokatheter koaxial einführen, durch den wiederum die Vorrichtung in die Zielregion gebracht wird. Den mit der Vorrichtung fixierten Thrombus wird man vorzugsweise bis in den Führungskatheter zurückziehen und dann mit diesem zusammen aus dem Körper entfernen.

Im Zusammenhang mit dieser Erfindung beziehen sich die Begriffe "distal" und "proximal" auf die Sicht vom behandelnden Arzt aus. Das distale Ende ist somit jeweils das dem behandelnden Arzt abgewandte Ende, was den weiter ins Blutgefäßsystem vorgeschobenen Bestandteilen der Vorrichtung entspricht, während proximal dem behandelnden Arzt zugewandt bedeutet, d. h. die proximal angeordneten Bestandteile der Vorrichtung sind weniger weit ins Blutgefäßsystem vorgeschoben.

Soweit in dieser Anmeldung von Längsrichtung die Rede ist, ist hierunter letztlich die Vorschubrichtung der Vorrichtung zu verstehen, d. h. die Längsachse der Vorrichtung entspricht auch der Längsachse des Blutgefäßes, innerhalb der die Vorrichtung vorgeschoben wird.

Bei der Herstellung der erfindungsgemäßen Vorrichtung geht man so vor, dass die Fasern, die später die Bürste bilden, nebeneinander und ggf. zusätzlich übereinander zwischen zwei Seelendrähte gelegt werden, wobei die Fasern orthogonal zu den Seelendrähten verlaufen. In diesem Zusammenhang sei angemerkt, dass unter einem orthogonalen Verlauf gemäß der Erfindung nicht lediglich ein Winkel von exakt 90° verstanden wird, sondern jeglicher Querverlauf der Fasern zu den Seelendrähten, d. h. die Fasern verlaufen im Wesentlichen quer zu den Seelendrähten, nicht parallel. Entsprechend können auch Winkel von beispielsweise 70° in diesem Zusammenhang noch als orthogonal aufgefasst werden. Wenn die Fasern zwischen die Seelendrähte eingelegt sind, werden diese miteinander verdrillt, beispielsweise indem ein Ende festgehalten wird, während das andere gedreht bzw. tordiert wird, um eine plastische Verformung der Seelendrähte zu einer Spiralstruktur herbeizuführen. Nach der Verdrillung der Seelendrähte stehen die Fasern gewissermaßen auf einer Schraubenlinie aus den verdrehten Seelendrähten nach außen hervor. Der wesentliche Vorteil einer solchen Vorrichtung liegt darin, dass relativ wenig Seelendraht verwendet werden muss, um gleichzeitig einen sehr hohen Faserbesatz des als Bürste dienenden distalen Elements zu erreichen. Die Verwendung von wenig Seelendraht ist insofern vorteilhaft, als das System dadurch besonders flexibel bleibt. Darüber hinaus ist die Festlegung der Fasern an den Seelendrähten in dieser Ausführungsform besonders einfach und die Verteilung der Fasern auf der Bürste ist sehr gleichmäßig.

Die Fasermenge bzw. -dichte lässt sich durch die Anzahl der Verdrehungen der Seelendrähte steuern, was zu unterschiedlichen Härtegraden bezogen auf die Radialkraft der Bürste führt, da durch mehr Verdrehungen auch mehr Fasern pro Längeneinheit der Bürste erzeugt werden. Darüber hinaus lässt sich die Biegesteifigkeit der Seele u. a. durch die Anzahl der Seelendrähte und der Verdrehungen einstellen. So wird beispielsweise durch eine hohe Anzahl von Verdrehungen von zwei oder mehr Seelendrähten eine Doppelhelix aus diesen beiden Seelendrähten ausgebildet, die weniger biegesteif ist als bei einer Ausführung mit weniger Umdrehungen.

Gegebenenfalls können die mindestens zwei Seelendrähte auch am distalen Ende miteinander verbunden sein und eine Schlaufe bilden. Letztlich handelt es sich somit um lediglich einen Seelendraht, der zunächst bis zum distalen Ende hin und anschließend wieder zurückgeführt wird, um schließlich die beiden parallel liegenden Abschnitte des Seelendrahtes mit dazwischen liegenden Fasern zu verdrillen.

Am proximalen Ende können die Seelendrähte über eine Wendel (Coil) mit weiteren, proximal gelegenen Komponenten der Vorrichtung verbunden sein. Bei diesen weiter proximal gelegenen Komponenten kann es sich insbesondere um einen Führungsdraht oder auch um weitere als Bürste ausgebildete distale Elemente handeln. Die Verbindung über derartige Coils ist in der Medizintechnik insbesondere aus dem Bereich der Occlusionswendeln für Aneurysmen hinlänglich bekannt. Die Verbindung der Coil mit den Seelendrähten kann dabei durch Verschweißen, Verkleben, Verlöten oder mechanische (d. h. kraft- und/oder formschlüssige) Fügeverfahren erfolgen.

Die Seelendrähte können aus Platin bzw. seinen Legierungen, insbesondere aus einer Platin-Iridiumlegierung, Edelstahl, Nickel-Titan-Legierungen wie Nitinol oder auch Wolfram und Wolframlegierungen sowie Kombinationen der genannten Materialien bestehen.

Die Seelendrähte und damit auch die gesamte Bürste können gerade ausgeführt sein oder auch eine Sekundärstruktur ausbilden, z. B wellen- oder helixförmig, d. h. die miteinander verdrillten Seelendrähte bilden wiederum eine Wellen- oder Helixform der Seele selbst aus. Der Unterschied zwischen Welle und Helix ist in diesem Zusammenhang so zu verstehen, dass eine Welle eine lediglich zweidimensionale, eine Helix hingegen eine dreidimensionale Form ausbildet. Die Wellen- oder Helixform der Bürste weist insofern Vorteile auf, als hiermit eine bessere Reinigungswirkung verbunden ist. Dabei kann auch der Durchmesser der Helix entweder von proximal nach distal oder von distal nach proximal zunehmen.

Im Falle einer durch die verdrillten Seelendrähte ausgebildeten Sekundärstruktur kann zusätzlich durch den Innenraum der Sekundärstruktur oder außerhalb ein Streckschutzfilament verlaufen, das eine axiale Streckung der Vorrichtung über die Nennlänge verhindert. Derartige Streckschutzfilamente können aus Polymermaterialien (z. B. Nylon) oder Metall, wie z. B. Nickel-Titanlegierungen (Nitinol) hergestellt sein. Dabei sind sie sowohl distal als auch proximal mit der durch die Seelendrähte ausgebildeten Sekundärstruktur verbunden. Auch das Streckschutzfilament selbst kann wiederum gerade, wellenförmig oder helixförmig beschaffen sein, wobei die zuletzt genannten Varianten einen gewissen Spielraum in Längsrichtung hinsichtlich einer möglichen axialen Streckung erlauben. Um noch einen hinreichenden Schutz gegenüber Streckung herbeizuführen, sollte eine solche Helix jedoch eine sehr hohe Steigung aufweisen, so dass sich der Spielraum der axialen Streckung in gewissen Grenzen hält.

Die erwähnten Seelendrähte müssen nicht in jedem Fall klassische, im Querschnitt runde Drähte sein, es kann sich auch um geschnittene oder geformte Formstücke handeln, beispielsweise wenn die Seelendrähte aus einer Platte herausgeschnitten werden. In diesem Fall ist der Querschnitt der Seelendrähte nicht rund sondern quadratisch oder viereckig.

Gemäß einer weiteren vorteilhaften Ausführungsform weist die Vorrichtung mehrere distale Elemente auf, von denen aus Fasern radial nach außen hervorstehen. Ein solches System bestehend aus mehreren Bürsten kann beispielsweise Vorteile aufweisen, wenn besonders große Thromben oder auch mehrere Thromben aus dem Blutgefäßsystem entfernt werden sollen. Darüber hinaus kann eine weiter distal gelegene Bürste ggf. Bruchstücke eines Thrombus, die von der proximalen Bürste abfallen, auffangen und mit entfernen.

Um trotz der Länge eines solchen System aus mehreren Bürstenteilen trotzdem noch eine ausreichende Flexibilität zu erreichen, ist es sinnvoll, die einzelnen distalen Elemente über Verbindungselemente, insbesondere Gelenke, miteinander zu verbinden. An diesem Gelenk kann die Vorrichtung in gewissem Maße abknicken und damit dem Verlauf der Blutgefäße folgen.

Gegebenenfalls können auch mehrere eine Bürste ausbildende distale Elemente, vorzugsweise zwei oder drei Bürsten, im Querschnitt der Vorrichtung betrachtet nebeneinander angeordnet werden, vorzugsweise parallel. Proximal und distal sind diese Bürsten wiederum miteinander verbunden, beispielsweise indem die Seelendrähte der Bürsten proximal und distal wieder zentral zusammenlaufen. Eine solche Ausführungsform ist mit dem Vorteil verbunden, dass jede einzelne Bürste über kürzere Fasern verfügen kann, da sich die Fasern nicht vom Zentrum der Vorrichtung bis zur Gefäßinnenwand erstrecken müssen, sondern lediglich von den einzelnen distalen Elementen aus, welche bei einer Anordnung nebeneinander den Gefäßwänden näher kommen. Da kürzere bzw. weniger weit aus dem distalen Element herausstehende Fasern letztlich härter sind, kann die Reinigungswirkung auf diese Weise weiter verbessert werden. Gegebenenfalls können die einzelnen Bürsten auch noch weiter zu einer Helix verdrillt sein.

Des Weiteren können die distalen Elemente mit Streben (zweckmäßigerweise mindestens 3) versehen sein, die vom distalen Ende des distalen Elementes ausgehen, radial nach außen hervortreten und am proximalen Ende des distalen Elementes wieder zentral zusammenlaufen. Die radiale Ausdehnung der Streben entspricht dabei vorzugsweise in etwa der radialen Ausdehnung der Fasern. Im mittleren Bereich verlaufen die Streben somit im Wesentlichen parallel zur innenliegenden Seele des distalen Elements. Das Vorsehen von Streben am distalen Element dient der zusätzlichen Führung des Systems im Blutgefäß. In gewissem Maße weisen die Streben eine Ähnlichkeit zu Korbstrukturen auf, allerdings müssen diese Streben nicht so dicht verlaufen, so dass u. U. nicht von einer echten Korbstruktur gesprochen werden kann.

In Fällen, in denen die Vorrichtung mehrere distale Elemente mit nach außen stehenden Fasern aufweist, können die Streben jeweils ein distales Element überspannen oder auch mehrere distale Elemente. Beispielsweise können bei zwei distalen Elementen die Streben vom distalen Ende des am weitesten distal gelegenen distalen Elements ausgehen und erst am proximalen Ende der proximalen Bürste wieder zusammenlaufen. In diesem Fall ist es sinnvoll, zusätzliche Zwischenstreben zwischen den Streben und den zentral verlaufenden Seelendrähten vorzusehen, um eine zusätzliche Stabilität der Strebenstruktur zu erzeugen.

Gemäß der Erfindung haben die Bürsten eine konische Struktur, d. h. die radiale Ausdehnung der Fasern, was letztlich dem Bürstendurchmesser entspricht, nimmt von proximal nach distal zu. Der wesentliche Vorteil einer solchen konischen Bürstenform liegt darin, dass unabhängig von der Weite des konkret zu reinigenden Blutgefäßes stets zumindest einige Bürstenbereiche vorhanden sind, bei denen die Fasern die optimale Länge aufweisen. Eine optimale Länge für ein bestimmtes Blutgefäß weisen Fasern gerade dann auf, wenn die Fasern an den Wänden des Blutgefäßes anliegen, ohne bei der Bewegung der Vorrichtung in Richtung proximal in distale Richtung verbogen zu werden. In diesem Fall ist die Reinigungswirkung der Fasern besonders gut. Längere Fasern hingegen werden bei der Rückwärtsbewegung in proximale Richtung in Richtung distal verbogen und reinigen daher nicht mehr so effektiv, kurze Fasern wiederum erreichen u. U. gar nicht die Gefäßinnenwand und bewirken daher ohnehin keine Reinigungswirkung. Dadurch, dass bei einer konischen Bürstenform nicht alle Fasern gleich lang sind, sind somit auch bei unterschiedlichen Blutgefäßen stets zumindest einige Fasern vorhanden, die die optimale Reinigungswirkung entfalten.

Vorgesehen ist ein konisches distales Element (Bürste), bei dem die radiale Ausdehnung der Fasern von proximal nach distal zunimmt. Dies läßt sich erreichen, indem von proximal nach distal Fasern mit zunehmender Länge zwischen die Seelendrähte eingelegt werden. Eine solche Bürste hat den Vorteil, dass die längeren distal liegenden Fasern ggf. einzelne Thrombusfragmente mit einfangen können, wenn die Vorrichtung nach proximal zurückgezogen wird.

Die konischen Bürsten können dabei unterschiedliche Formen aufweisen, ausreichend ist grundsätzlich eine konische Bürste. Es können jedoch auch mehrere konische Bürsten vorgesehen sein, die über ein Gelenk miteinander verbunden sind, oder auch viele kurze konische Abschnitte an einem einzelnen distalen Element.

Zusätzlich können auch die Fasern im proximalen Bereich eines distalen Elements härter ausgebildet sein als im distalen Bereich. Die härteren Fasern im proximalen Bereich dienen dabei mehr dem Abschaben eines an der Gefäßwand haftenden Thrombus, während die weicheren Fasern im distalen Bereich mehr dem Festhalten des Thrombus bzw. von Thrombusfragmenten dienen.

Um die Aufnahme eines Clots (Thrombus) zu erleichtern, kann die Bürste des distalen Elements entlang der Seele unterschiedlich gestaltet sein. Beispielsweise können einige Bereiche, insbesondere der mittlere Bereich einer Bürste, mit weicheren Fasern besetzt sein, wobei die Fasern der Aufnahme des Clots dienen. Die mit härteren Fasern besetzten weiter proximal bzw. distal gelegenen Bereiche hingegen sollen eher eine verstärkte Reinigungswirkung hervorrufen.

Einen ähnlichen Effekt hat es, wenn die Dichte des Faserbesatzes in einigen Bereichen, insbesondere im mittleren Bereich des distalen Elements geringer ist als in anderen Bereichen. Auch dies hat wiederum den Effekt, dass die dichter besetzten proximalen und distalen Bereiche der Bürste eher der Verbesserung der Reinigungswirkung an den Gefäßwänden dienen, während der mittlere, dünner besetzte Bereich den Clot aufnehmen soll.

Die Fasern werden zwischen den miteinander verdrillten Seelendrähten in erster Linie dadurch festgehalten, dass sie beim Verdrillen eingeklemmt werden. Zusätzlich oder alternativ kann jedoch eine weitere Festlegung durch Verkleben, Verknoten und/oder Anschmelzen erfolgen.

Die radial außenliegenden Enden der Fasern weisen vorteilhafterweise Verdickungen auf, beispielsweise kugelförmige Verdickungen, um so der Clotmasse mehr Oberfläche und Halt zur Verfügung zu stellen. Ein weiterer Vorteil dieser Ausführungsform besteht darin, dass die Faserenden auf diese Weise atraumatisch gestaltet werden können. Die Verdickungen an den Faserenden lassen sich beispielsweise dadurch erzielen, dass das Trennen der Fasern mittels Mikrolaserschneiden, Elektronenstrahlschneiden o.ä. erfolgt.

Gemäß einer weiteren Ausführungsform sind die radial außen liegenden Enden der Fasern zumindest teilweise über Schlaufen miteinander verbunden. Letztlich handelt es sich bei den miteinander verbundenen Fasern somit nicht um zwei sondern lediglich um eine Faser mit einem schlaufenförmigen Verlauf. Die Faser tritt aus der Seele hervor, verläuft bis zum äußeren Bürstenrand, bildet eine Schlaufe aus und verläuft wieder zur Seele zurück. Insgesamt ergibt sich somit ein elliptischer Verlauf der Faser. Diese Ausführungsform hat den Vorteil, dass, ähnlich wie bei der Verdickung am Faserende, der Clotmasse mehr Oberfläche zur Verfügung gestellt wird, um auf diese Weise die Einfangwirkung für den Thrombus zu verbessern. Darüber hinaus ist die Schlaufe abgerundet und damit atraumatisch. Ein weiterer Vorteil besteht darin, dass die Fasern steifer werden, da die schlaufenförmig verlaufende Faser letztlich ein Verhalten wie zwei nebeneinander verlaufende Fasern aufzeigt.

Gemäß einer weiteren Ausführungsform verlaufen die Seelendrähte im Querschnitt des distalen Elements außerhalb des Zentrums, d. h. exzentrisch. Eine solche Bürste mit exzentrisch angeordneter Seele bewirkt, dass auf einer Seite der Bürste vergleichsweise kurze, auf der anderen Seite der Seele lange Fasern vorhanden sind. Aufgrund ihres kurzen Abstands zur Seele verhalten sich dabei die kurzen Enden der Fasern deutlich härter, die langen Enden der Fasern deutlich weicher, wobei wiederum die härteren Fasern eher einer Verbesserung der Reinigungswirkung dienen, die weicheren Fasern eher dem Festhalten des Thrombus. Ein weiterer Vorteil einer Bürste mit exzentrisch angeordneter Seele kann darin liegen, dass eine solche Bürste leichter seitlich an einem Clot vorbei geführt werden kann, um ihn anschließend bei der Rückwärtsbewegung in proximaler Richtung durch die Bürste aufzunehmen.

Die beschriebenen bürstenartigen Vorrichtungen können auch grundsätzlich zur Verfüllung von Aneurysmen verwendet werden. In diesem Fall sollte es sich bei den Bürsten um helixförmige Bürsten handeln, die im Vergleich zu solchen Bürsten, die der Thrombusentfernung dienen, vergleichsweise kurz sind. Um die Bürsten im Aneurysma platzieren zu können, muss das bzw. die distalen Elemente vom Führungsdraht abtrennbar ausgebildet sein. Um das Aneurysma weiter auszufüllen, können die Borsten auch mit einem Kleber versehen sein oder eine thrombogene Beschichtung aufweisen.

Die oben beschriebene Konusform der Bürste, bei der der Durchmesser des distalen Elements von proximal nach distal zunimmt, hat sich als besonders vorteilhaft herausgestellt.

Gemäß einer alternativen Ausführungsform (unabhängig von der zuvor beschriebenen Bürstenstruktur) wird eine Vorrichtung mit einem Führungsdraht zur Entfernung von Fremdkörpern und Thromben aus Körperhohlräumen und Blutgefäßen zur Verfügung gestellt, wobei die Vorrichtung eine Korbstruktur am distalen Ende des Führungsdrahtes aufweist, die sich aus einzelnen Streben zusammensetzt und geeignet ist, sich unter dem äußeren Zwang eines Mikrokatheters eng zusammenzufalten und innerhalb des Mikrokatheters transportiert zu werden, und sich bei Wegfall des äußeren Zwangs durch den Mikrokatheter zur vollen Korbstruktur entfaltet, wobei die Korbstruktur mindestens zwei in Längsrichtung hintereinander angeordnete Körbe umfasst.

Eine solche Vorrichtung zur Entfernung von Thromben mit Korbstruktur ist grundsätzlich dazu vorgesehen, Thromben im Inneren aufzunehmen und auf diese Weise die Entfernung von Thromben zu ermöglichen. Im zusammengefalteten Zustand wird die Korbstruktur innerhalb eines Mikrokatheters durch die Blutgefäße bis an den Zielort gebracht und dort schließlich distal des zu entfernenden Thrombus aus dem Mikrokatheter hinausgeschoben, woraufhin sich die Vorrichtung zur vollen Korbstruktur entfaltet. Die Korbstruktur kann die zu entfernenden Thromben vollständig oder in zerkleinerter Form aufnehmen. Anschließend wird die Vorrichtung im Blutgefäßsystem zurückgezogen und schließlich, gegebenenfalls nach Einziehen in einen Katheter, bei dem es sich um einen zusätzlichen Führungskatheter (guide catheter) handeln kann, der einen größeren Innendurchmesser aufweist als der Mikrokatheter, durch den die Korbstruktur an ihren Zielort gebracht wurde, zusammen mit dem Thrombus gänzlich aus dem Blutgefäßsystem entfernt.

Die Korbstruktur weist im Allgemeinen eine längliche, schiffähnliche Struktur mit einer Länge von 5 bis 50 mm und einen Durchmesser von 2 bis 6 mm im expandierten Zustand auf. Die Korbstruktur wird aus peripheren, insbesondere längs verlaufenden Streben gebildet. Diese Streben sind üblicherweise regelmäßig über den Umfang verteilt.

Erfindungsgemäß weist diese Korbstruktur mindestens zwei in Längsrichtung hintereinander angeordnete Körbe auf, so dass es sich um eine Doppelkorbstruktur handelt. Zwei oder mehr hintereinander angeordnete Körbe haben dabei den Vorteil, dass diese letztlich unterschiedlichen Zwecken dienen können. So kann beispielsweise der proximal angeordnete Korb als Trennwerkzeug für den Thrombus bzw. im Gefäß gebildeten Clot wirken, während der distale Korb den Thrombus oder die Bruchstücke des Thrombus aufnehmen kann. Eine solche Zerteilung des Clots hat insbesondere dann Bedeutung, wenn der Clot insgesamt zu groß ist, als im Ganzen von der Korbstruktur aufgenommen werden können, oder aber so stark verhärtet ist, dass er nur schwer in die Korbstruktur hineingelangt. Eine solch starke Verhärtung des Clots ist vermutlich insbesondere bei älteren Thromben festzustellen.

Die Korbstruktur wird, wie bereits erwähnt, aus Streben gebildet, die vorzugsweise mindestens zum Teil in Längsrichtung verlaufen. Hierbei ist zu beachten, dass unter in Längsrichtung verlaufenden Streben nicht nur solche verstanden werden, die exakt parallel zur Längsachse ausgerichtet sind, sondern auch solche, die in einem gewissen Winkel < 90° zur Längsachse in Richtung distal bzw. proximal verlaufen. Um von einer echten Korbstruktur sprechen zu können, wird diese vorzugsweise aus mindestens drei, besser jedoch vier bis acht Streben gebildet.

Weiterhin ist es zweckmäßig, wenn die Korbstruktur und somit die Streben aus einem Formgedächtnismaterial, vorzugsweise Nitinol, bestehen, so dass sie sich im zusammengefalteten Zustand in einem Mikrokatheter transportieren läßt und nach Herausschieben aus dem Mikrokatheter selbsttätig auffaltet.

Die Entfaltung zur vollen Korbstruktur bei Wegfall des äußeren Zwangs durch den Mikrokatheter muss jedoch nicht in jedem Fall selbsttätig erfolgen, sondern kann auch manuell herbeigeführt werden. Beispielsweise könnte an der Korbstruktur ein zusätzlicher Führungsdraht angebracht sein, der beim Vorschieben eine Entfaltung der Korbstruktur bewirkt.

Zentral durch die Korbstruktur verläuft zweckmäßigerweise ein Kernfilament. Bei diesem Kernfilament handelt es sich um den distalen Abschnitt des Führungsdrahtes oder auch um ein gesondertes Kernfilament, das den Führungsdraht in Richtung distal fortsetzt.

Die Zusammenfaltung der Korbstruktur unter dem äußeren Zwang eines Katheters ist im Allgemeinen mit einer Streckung der Korbstruktur verbunden. Um diese Streckung aufzufangen bzw. bei der Freisetzung der Korbstruktur aus dem Katheter die radiale Expansion, die mit einer Längskontraktion verbunden ist, zu erleichtern, ist es sinnvoll, die Korbstruktur so beweglich auszugestalten, dass sie dieser Streckung/Kontraktion folgen kann. Zumindest an ihrem proximalen Ende sollte sich daher die Korbstruktur relativ zum Kernfilament verschieben lassen. Dies kann dadurch erreicht werden, dass die Korbstruktur an ihrem proximalen Ende in einer Hülse zusammenläuft, durch welche das Kernfilament in Längsrichtung beweglich ist.

Da bei der erfindungsgemäßen Doppelkorbstruktur der weiter proximal angeordnete Korb in erster Linie als Trennwerkzeug für den zu entfernenden Clot dienen soll, während der distale Korb zur Aufnahme des Clots vorgesehen ist, ist es sinnvoll, die Körbe ihren konkreten Aufgaben entsprechend auszugestalten. So kann etwa der distal angeordnete Korb länger ausgebildet werden, um so ausreichend Raum für den aufzunehmenden Thrombus zur Verfügung zu stellen. Der der Auftrennung des Thrombus dienende proximale Korb hingegen kann in Längsrichtung kürzer ausgestaltet werden.

Da mit dem proximal angeordneten Korb letztlich Kräfte auf den Thrombus ausgeübt werden sollen, die zur Zerkleinerung des Thrombus führen, ist es darüber hinaus sinnvoll, diesen Korb härter auszugestalten als den distal angeordneten Korb. Eine härtere Ausgestaltung ist in diesem Zusammenhang so zu verstehen, dass die zur Verformung erforderlichen Radialkräfte größer sind als die Radialkräfte, die zur Verformung des distal angeordneten Korbs benötigt werden. Durch eine entsprechend harte Ausgestaltung des proximal angeordneten Korbs können auch sehr alte, bereits stark verhärtete Thromben soweit zerkleinert oder auch von den Gefäßwänden abgeschabt werden, dass sie anschließend nach Aufnahme im distal angeordneten Korb aus dem Blutgefäß entfernt werden können.

Um den proximal und distal angeordneten Korb an ihre entsprechenden Aufgaben anzupassen, können sie aus unterschiedlichen Materialien gefertigt sein, wobei, wie oben ausgeführt, für den proximalen Korb ein härteres Material verwendet werden sollte. Beispielsweise können hier unterschiedliche Nitinol-Werkstoffe verwendet werden, d. h. insbesondere unterschiedliche Legierungen.

Alternativ oder zusätzlich können auch die Streben, aus denen der proximal angeordnete Korb gebildet ist, einen größeren Durchmesser aufweisen als die Streben, aus denen der distal angeordnete Korb besteht. Auch auf diese Weise wird erreicht, dass der proximale Korb größeren Radialkräften zu widerstehen in der Lage und somit letztlich härter ist.

Eine weitere Möglichkeit besteht in der Wahl des Winkels, in dem die Streben zur Längsachse stehen. So können die Streben, aus denen der proximal angeordnete Korb gebildet ist, zumindest teilweise in einem größeren Winkel zur Längsachse verlaufen als die Streben des distal angeordneten Korbes, was bewirkt, dass der proximal angeordnete Korb durch Radialkräfte weniger leicht zusammenstauchbar ist. insgesamt hat somit der distal angeordnete Korb eine etwas flachere Struktur, die insbesondere dann ins Auge fällt, wenn zusätzlich der distale Korb länger ausgestaltet ist als der proximale Korb.

In Fällen, in denen besonders viel Clot-Material aufgenommen werden soll, etwa weil der Thrombus besonders groß ist, können auch Korbstrukturen verwendet werden, die mehr als zwei Körbe umfassen. In diesem Fall dienen sämtliche Körbe distal des proximal angeordneten Korbs zur Aufnahme des Clot-Materials.

Wie bereits erwähnt, können die Körbe aus unterschiedlich vielen Streben gebildet werden, wobei letztlich der Korb umso dichter ist, desto mehr Streben die Korbstruktur aufweist. Je nach Einsatzzweck hat sich allerdings herausgestellt, dass es sinnvoll sein kann, nicht zu viele Streben zu verwenden, um die proximale Öffnung der Korbstruktur, durch die der Thrombus in die Korbstruktur eindringen soll, nicht zu sehr zu verkleinern. So werden letztlich die zwischen den Streben liegenden Teilöffnungen im proximalen Querschnitt der Korbstruktur betrachtet umso größer, desto weniger Streben verwendet werden. Dies ist insbesondere dann wichtig, wenn der Thrombus besonders alt und somit fest und zusammenhängend ist, so dass dieser nur noch schwer in die Öffnung der Korbstruktur gelangt. Für derartige Thromben hat sich eine Zahl der Streben von vier bis sechs als besonders vorteilhaft herausgestellt.

Selbstverständlich sind hierbei auch Kombinationen denkbar, etwa in der Form, dass der proximale Korb, in den der Thrombus beim Zurückziehen der Korbstruktur im Blutgefäßsystem zunächst eindringen muß, eine geringere Anzahl von Streben aufweist als der distal angeordnete Korb, der für die Aufnahme des bereits durch den proximalen Korb zerkleinerten Thrombus vorgesehen ist.

Üblicherweise laufen die Streben jedes Korbs jeweils am distalen und am proximalen Ende des Korbs in einem Punkt zusammen, wobei dieser Punkt vorzugsweise zentral angeordnet ist. An den Punkten, an denen die Streben zusammenlaufen, können die Streben unmittelbar oder mittelbar über ein Verbindungselement miteinander verbunden sein. Gegebenenfalls kann das Verbindungselement als Hülse ausgebindet sein, die gegenüber einem zentral verlaufenden Kernfilament längs beweglich ist, um auf diese Weise Streckung und Kontraktion der Korbstruktur beim Ein- bzw. Ausschieben aus dem Katheter zu erleichtern.

Ein anderer Ansatz, die proximale Öffnung der Korbstruktur zu vergrößern, besteht neben der oben beschriebenen Möglichkeit der Verringerung der Anzahl der Streben darin, die zwischen den Streben sich ergebenden Teilöffnungen zumindest teilweise zu vergrößern, um auf diese Weise das Eindringen eines fest zusammenhängenden Thrombus zu erleichtern. Hierzu werden die zwischen den Streben bestehenden großen Teilöffnungen vergrößert, womit sich gleichzeitig eine Verkleinerung der kleinen Teilöffnungen ergibt. Dies ist dadurch zu erreichen, dass die Streben mindestens eines Korbes, vorzugsweise des proximal angeordneten Korbs, bei entfalteter Korbstruktur von dem Punkt aus, in dem die Streben am proximalen Ende des Korbs zusammenlaufen, entlang eines ersten Abschnitts gruppiert, z. B. paarweise eng benachbart in Richtung distal verlaufen, distal dieses ersten Abschnitts auseinander laufen und sich in einem zweiten Abschnitt distal des ersten Abschnitts entlang des Umfangs des Korbs gleichmäßig verteilt in Richtung distal erstrecken. Mit anderen Worten verlaufen die Streben nicht entlang des gesamten Korbs gleichmäßig über den Umfang verteilt, vielmehr wird diese gleichmäßige Verteilung erst ein Stück distal der proximalen Öffnung hergestellt. Dadurch, dass die Streben am proximalen Ende des Korbs zunächst gruppiert eng benachbart verlaufen, werden Teilöffnungen geschaffen, die in etwa so groß sind, als wäre nur die halbe oder ein Drittel der Anzahl an Streben zum Einsatz gekommen. Bei der Verwendung von insgesamt vier Streben etwa verlaufen jeweils etwa zwei Strebenpaare an der proximalen Öffnung zusammen, so dass sich zwei erheblich vergrößerte Teilöffnungen ergeben, die nahezu halbkreisförmig sind, während die verbleibenden kleinen Teilöffnungen zwischen den eng benachbart verlaufenden Streben zu vernachlässigen sind. In entsprechender Weise ergäben sich bei insgesamt sechs Streben und zunächst paarweisem Verlauf der Streben Teilöffnungen, die nahezu die Größe eines Drittelkreises haben. Wenn entlang des ersten Abschnitts jeweils drei Streben eng benachbart in Richtung distal verlaufen, ergeben sich sogar bei der Verwendung von sechs Streben zwei nahezu halbkreisförmige Öffnungen. Grundsätzlich ist ein derartiger Ansatz zur teilweisen Vergrößerung der Teilöffnungen selbstverständlich auch bei Korbstrukturen denkbar, die lediglich aus einem Korb bestehen.

Die die Körbe bildenden Streben können für den proximalen und für den distalen Korb identisch sein. In diesem Fall erstrecken sich die Streben vom proximalen Ende des proximal angeordneten Korbs bis zum distalen Ende des distal angeordneten Korbs. Damit die Streben dabei eine Doppelkorbstruktur bilden und nicht lediglich einen stark verlängerten Korb ausbilden, werden sich die Streben dabei vorzugsweise zumindest teilweise überkreuzen, so dass letztlich der Knotenpunkt, in dem sich die Streben treffen, das distale Ende des proximal angeordneten Korbs und gleichzeitig das proximale Ende des distal angeordneten Korbs darstellen. Eine derartige Ausführungsform hat den Vorteil, dass sich die auf den distalen Korb und die auf den proximalen Korb einwirkenden Radialkräfte gegenseitig beeinflussen, da radiale Kräfte in den Streben auf den anderen Korb übertragen werden. Auf diese Weise kann die Vorrichtung ihre aufgespannte Form beibehalten, auch wenn der Nenndurchmesser in einem Gefäß nicht realisiert werden kann. Dies gilt insbesondere dann, wenn die weiter oben beschriebenen Vorkehrungen zur Längenanpassung der Korbstruktur gegeben sind.

Im Knotenpunkt können die Streben untereinander und/oder mit dem Kernfilament verbunden sein. Vorteilhafterweise besteht jedoch keine Verbindung, da auf diese Weise die Radialkraftbeeinflussung nicht gestört wird.

Darüber hinaus können die radialen Kräfte durch nicht ganz vollständiges Ausschieben der Vorrichtung aus dem Mikrokatheter beeinflusst werden. Wenn beispielsweise nur der distale Korb ausgeschoben wird, während der proximale im Mikrokatheter verbleibt, erhöht sich die Radialkraft des distal angeordneten Korbs. Dies kann bei der Bergung besonders fester Clots/Thromben von Vorteil sein.

Besonders gut ist die gegenseitige Beeinflussung der Radialkräfte dann, wenn die Streben vom proximal angeordneten Korb zum nächst gelegenen distal angeordneten Korb auf die gegenüberliegende Seite der Korbstruktur verlaufen, d. h., wenn sich von einem Korb zum nächsten ein Versatz um 180° ergibt.

Der am weitesten distal angeordnete Korb kann zweckmäßigerweise zumindest distal eine Netzstruktur aufweisen, um auf diese Weise eine verbesserte Sicherheit gegen ein Herausrutschen von Thrombusfragmenten aus dem Korb beim Zurückführen der Vorrichtung aus dem Blutgefäß zu gewährleisten. Diese Netzstruktur kann sich insbesondere aus miteinander verflochtenem Draht, für den, ebenso wie für die Streben, Nitinol verwendet werden kann, zusammensetzen. Eine solche Netzstruktur eines Korbes ist insbesondere am distalen Ende sinnvoll, da zur Entfernung des Thrombus letztlich die Korbstruktur in Richtung proximal gezogen wird, während eine Netzstruktur am proximalen Ende des Korbes weniger hilfreich ist, da diese ansonsten unter Umständen das Eindringen des Thrombus in den Korb verhindern könnte. Entsprechend sollte sich die Netzstruktur lediglich am distalen Ende des Korbes befinden oder aber sich von proximal zu distal hin verdichten. Darüber hinaus erleichtert eine größere Maschenweite im proximalen Bereich des Korbs das Zurückziehen des Korbs in den Katheter unter Zusammenfaltung der Strukturen bei Einschluss des Thrombus.

Eine ähnliche Funktion, wie die geschilderte Netzstruktur, kann auch eine Bespannung des distal angeordneten Korbs auf seiner distalen Seite mit einer Polymerhaut übernehmen. Eine solche Polymerhaut, die beispielsweise aus expandiertem PTFE (Polytetrafluorethylen) bestehen kann, bewirkt praktisch, dass das distale Ende des Korbs zu einer Tasche wird. Die Polymerhaut erstreckt sich dabei von der distalen Spitze des Korbs entlang der Streben zu einer gewünschten Position, beispielsweise etwa in der Mitte des Korbs. Die Polymerhaut kann am distalen Ende des Korbs eine oder mehrere Öffnungen aufweisen, die so groß sind, dass Flüssigkeit, insbesondere Blut, ungehindert hindurch treten kann, während der mit der Korbstruktur eingefangene Thrombus oder Thrombusfragmente zurückgehalten werden. Dies ist insbesondere beim Zurückziehen der Korbstruktur von Vorteil, da auf diese Weise der Blutfluss selbst nicht behindert wird, der Thrombus aber dennoch restlos aus dem Blutgefäßsystem entfernt wird.

Gemäß einer weiteren vorteilhaften Ausführungsform verlaufen die Streben der Korbstruktur schraubenlinienförmig, d. h. distales Ende und proximales Ende sind gegeneinander um 45 bis 180° versetzt, vorzugsweise um etwa 90°. Ein solcher Schraubenlinienverlauf erlaubt es, beim Vorschieben der Korbstruktur einen an der Gefäßwand haftenden Thrombus abzuscheren bzw. abzuschneiden, ohne dass dazu die Vorrichtung gedreht werden müßte.

Gemäß einer anderen Variante verlaufen die Streben entlang einer Wellenlinie mit einer seitlichen Auslenkung um 45 bis 90°, d. h. die Streben bewegen sich zunächst seitlich, bis sie beispielsweise einen Punkt erreicht haben, der um 90° gegen den Startpunkt versetzt ist, und bewegen sich anschließend auf der zweiten Hälfte ihrer Länge zum Ausgangspunkt.zurück. In diesem Fall sind das proximale Ende und das distale Ende nicht gegeneinander versetzt.

Gemäß einer besonders bevorzugten Ausführungsform besteht die Vorrichtung nicht nur aus einer Korbstruktur am distalen Ende des Führungsdrahtes, sondern weist zusätzlich im Bereich der Korbstruktur radial nach außen stehende Fasern oder Borsten auf, die einzeln oder in Bündeln angeordnet sein können.

Die Fasern oder Borsten werden auf an sich bekannte Art und Weise mit einem distalen Element des Führungsdrahtes verbunden, wie dies beispielsweise aus der Fertigung von faserbewehrten Embolisationsspiralen bekannt ist. Dies kann durch Verkleben, Verschweißen oder jede andere geeignete Befestigungsart geschehen.

Vorteilhafterweise weist die Vorrichtung einen oder mehrere radiopake (röntgendichte) Marker auf. Diese können beispielsweise aus Platin oder einer Platinlegierung hergestellt sein. Derartige röntgendichte Marker können sich sowohl in den Bereichen befinden, von denen die Fasern oder Borsten ausgehen, aber auch an der Korbstruktur, damit der behandelnde Arzt mit entsprechenden bildgebenden Verfahren den Fortgang der Behandlung beobachten kann.

Es ist weiterhin vorteilhaft, wenn die Spitze der gesamten Vorrichtung atraumatisch, z. B. abgerundet, ausgebildet ist.

Das distale Element der Vorrichtung, das die Befaserung aufweist, verläuft sinnvollerweise zentral in der Korbstruktur, d. h. das Element mit seiner Befaserung befindet sich letztlich zentral innerhalb der Körbe. Auf diese Weise werden die vorteilhaften Wirkungen der Korbstruktur einerseits und der Fasern am distalen Element andererseits miteinander kombiniert. Dies ermöglicht ein besonders sicheres Festhalten des eingefangenen Thrombus sowohl durch die Korbstruktur als auch durch die Befaserung und ermöglicht auch das Zurückziehen des Thrombus in einen Katheter.

Die Fasern oder Borsten können sich insbesondere an einem oder mehreren auf dem durch die Korbstruktur verlaufenden Kernfilament befindlichen verschiebbaren Filamenten befinden und daran festgelegt sein, so dass die Fasern/Borsten praktisch vom Zentrum der Korbstruktur aus strahlförmig radial vorstehen. Das verschiebbare Filament kann z. B. ein schraubenförmig gewickelter Draht sein. Zusätzlich können auch Fasern oder Borsten an Streben der Korbstruktur festgelegt sein.

Des Weiteren sei angemerkt, dass auch hinsichtlich der Ausführungsform der Erfindung mit einer Korbstruktur zu den Fasern sämtliche Ausführungen gelten, die bereits zuvor im Zusammenhang mit dem bürstenförmigen distalen Element gemacht wurden, insbesondere hinsichtlich des Materials, das für die Fasern verwendet wird, der Winkelausrichtung, der Länge der Fasern, der Beschichtung der Fasern, der Verwendung von röntgendichten Markern etc.

Darüber hinaus sind sämtliche beschriebenen Ausführungsformen der Erfindung miteinander kombinierbar. Insbesondere kann die Ausführungsform mit der Doppelkorbstruktur zusätzliche Fasern aufweisen, umgekehrt kann die Ausführungsform mit vom distalen Element ausgehenden Fasern zusätzlich eine Korbstruktur haben. Insbesondere ist auch eine Kombination von Doppelkorbstruktur und distalem Element aus miteinander verdrillten Seelendrähten mit nach außen vorstehenden Fasern möglich.

Die Erfindung betrifft schließlich auch die Kombination der Vorrichtung mit einem Führungs- und/oder Mikrokatheter, in dem die Vorrichtung an den Einsatzort manövriert und mit dem Thrombus beladen wieder aus dem Blutgefäßsystem entfernt werden kann. Es kann sinnvoll sein, den Katheter dazu zusätzlich als Aspirationskatheter auszulegen, mit dem Mikrokatheter aufgenommen werden können.

Besondere Bedeutung hat die zuvor beschriebene Erfindung für die Entfernung von Thromben aus besonders kleinlumigen Gefäßen, insbesondere intrakraniell. Selbstverständlich läßt sich die Erfindung jedoch auch bei der Entfernung von Thromben aus anderen Bereichen des Körpers einsetzen, beispielsweise aus dem Herzen, der Lunge, den Beinen etc. Denkbar ist auch der Einsatz zum Entfernen sonstiger Fremdkörper aus den Blutgefäßen, beispielsweise von Embolisationsspiralen und Stents.

Die Erfindung wird nachfolgend beispielhaft anhand der beigefügten Zeichnungen näher erläutert. Es zeigen:
- Figur 1: die Herstellung eines bürstenartigen distalen Elements;
- Figur 2a: das distale Element im Längsschnitt;
- Figur 2b: das distale Element im Längsschnitt;
- Figur 2c: das distale Element im Querschnitt;
- Figur 3: eine Ausführungsform mit konischer Bürste
- Figur 4: eine weitere Ausführungsform des distalen Elements im Längsschnitt;
- Figur 5: ein distales Element mit wellenförmig verlaufender Seele;
- Figur 6: eine helixförmig verlaufende Seele;
- Figur 7: eine helixförmig verlaufende Seele mit nach außen hervorstehenden Fasern;
- Figur 8: eine Ausführungsform mit zwei hintereinander angeordneten distalen Elementen;
- Figur 9: eine Ausführungsform mit zwei hintereinander angeordneten distalen Elementen und zusätzlichen Streben;
- Figur 10: eine weitere Ausführungsform mit zwei hintereinander angeordneten distalen Elementen mit zusätzlichen Streben;
- Figur 11: eine Ausführungsform mit konischen Bürstenelementen;
- Figur 12: eine weitere Ausführungsform mit konischen Bürstenelementen;
- Figur 13: eine Ausführungsform mit Fasern unterschiedlicher Härte;
- Figur 14: eine Ausführungsform mit unterschiedlich dichtem Faserbesatz;
- Figur 15a: eine Ausführungsform mit exzentrisch angeordneter Seele im Längsschnitt;
- Figur 15b: eine Ausführungsform mit exzentrisch angeordneter Seele im Querschnitt;
- Figur 16: eine Ausführungsform mit Korbstruktur und Bürstenelement;
- Figur 17a: eine Darstellung einer Doppelkorbstruktur in der Seitenansicht;
- Figur 17b: die Doppelkorbstruktur aus Figur 17a in einer Ansicht von distal;
- Figur 18a: eine weitere Seitenansicht einer Doppelkorbstruktur;
- Figur 18b: die Ansicht der Doppelkorbstruktur aus Figur 18a von distal;
- Figur 19a: die proximale Ansicht einer Korbstruktur gemäß einer Ausführungsform;
- Figur 19b: die proximale Ansicht einer Korbstruktur gemäß einer alternativen Ausführungsform;
- Figur 20a: die proximale Ansicht einer Korbstruktur gemäß einer weiteren Ausführungsform;
- Figur 20b: die proximale Ansicht einer Korbstruktur gemäß einer weiteren Ausführungsform;
- Figur 20c: die proximale Ansicht einer Korbstruktur gemäß einer weiteren Ausführungsform;
- Figur 21: die Seitenansicht der Korbstruktur aus Figur 20a;
- Figur 22: die Seitenansicht einer weiteren Ausführungsform der Erfindung.

Die Vorrichtungen gemäß der vorliegenden Erfindung weisen ein distales Element auf, das mindestens zwei miteinander verdrillte Seelendrähte umfasst, zwischen denen Fasern quer zum Verlauf der Seelendrähte eingelegt und mit den Seelendrähten verdrillt sind, so dass die Fasern vom distalen Element radial nach außen hervorstehen, wobei die radiale Ausdehnung der Fasern von proximal nach distal zunimmt, auch wenn dies in den folgenden Ausführungsbeispielen nicht oder anders dargestellt oder beschrieben ist.

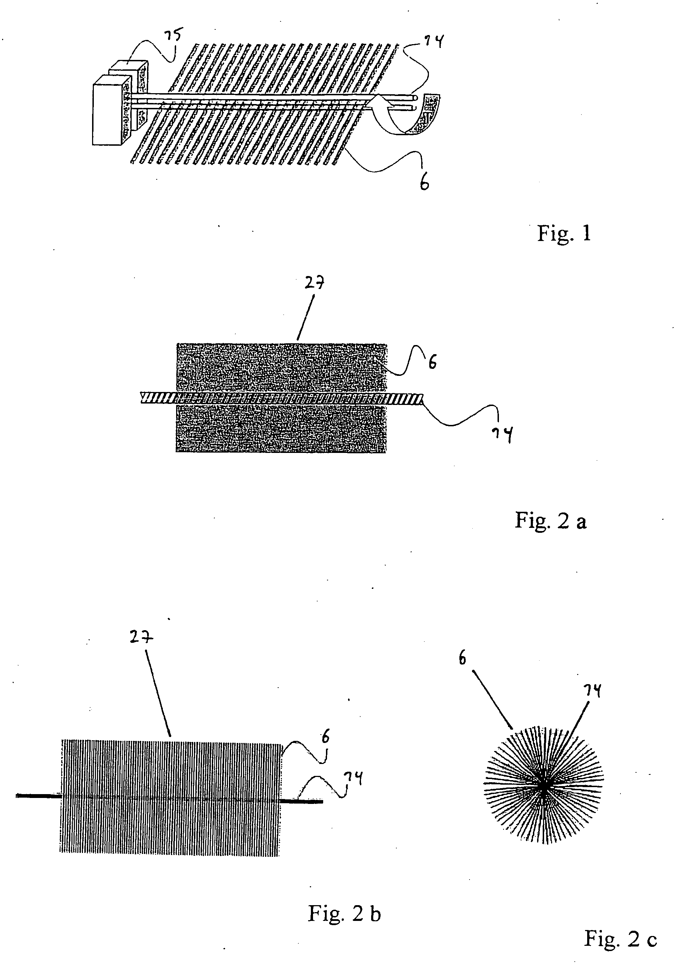

In Figur 1 ist die Erfindung dargestellt, bei der zwei Seelendrähte 14 parallel zueinander angeordnet werden, wobei zwischen den beiden Seelendrähten 14 orthogonal Fasern 6 verlaufen. Anschließend werden die Seelendrähte 14 an der Stelle 15 festgehalten und durch eine Torsionsbewegung T miteinander verdrillt. Auf diese Weise erhält man ein bürstenartiges distales Element 27, aus dem Fasern 6 radial nach außen hervor stehen.

Nach Verdrillung der Seelendrähte 14 miteinander hat das distale Element 27 ein Aussehen, wie es aus den Figuren 2a bis 2c hervorgeht, wobei es sich bei den Figuren 2a und 2b um Längsschnitte, bei der Figur 2c um eine Betrachtung von proximal oder distal handelt. Man erkennt, dass die Fasern 6 gleichmäßig entlang des Umfangs des distalen Elements 27 verteilt sind und radial nach außen hervor stehen.

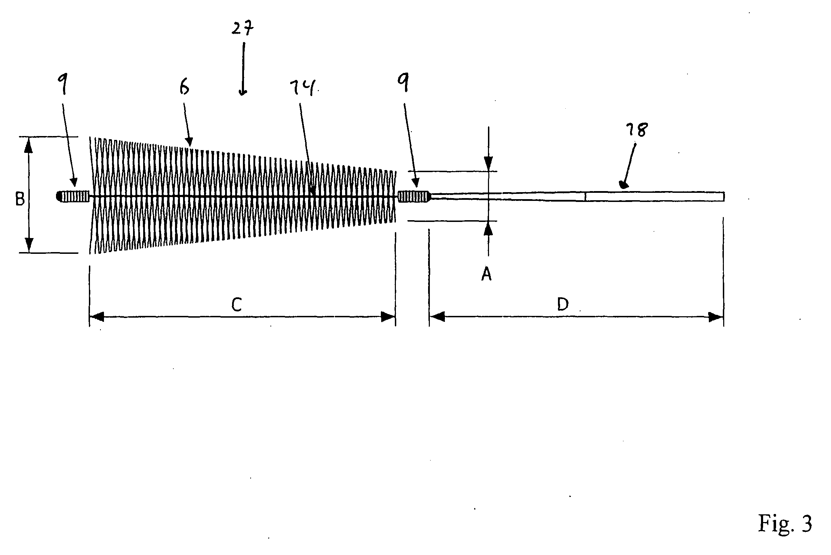

In Figur 3 ist eine Ausführungsform dargestellt, bei der das bürstenartige distale Element 27 konisch ausgebildet ist, d. h. von proximal nach distal im Durchmesser zunimmt. Der proximale Durchmesser A liegt typischerweise in einem Bereich von 1 bis 3 mm, der distale Durchmesser B zwischen 2 und 5 mm. Die Länge des distalen Elements 27 beträgt 10 bis 20 mm, die Länge D des Führungsdrahtes 18 beispielsweise 3000 mm. In der Regel wird die Seele 14 aus miteinander verwundenen Seelendrähten bestehen, denkbar sind jedoch auch konische Bürstenformen, bei denen die Fasern 6 auf andere Weise an der Seele 14 festgelegt sind. Des Weiteren verfügt die Vorrichtung proximal und distal des distalen Elements 27 über röntgendichte Marker 9.

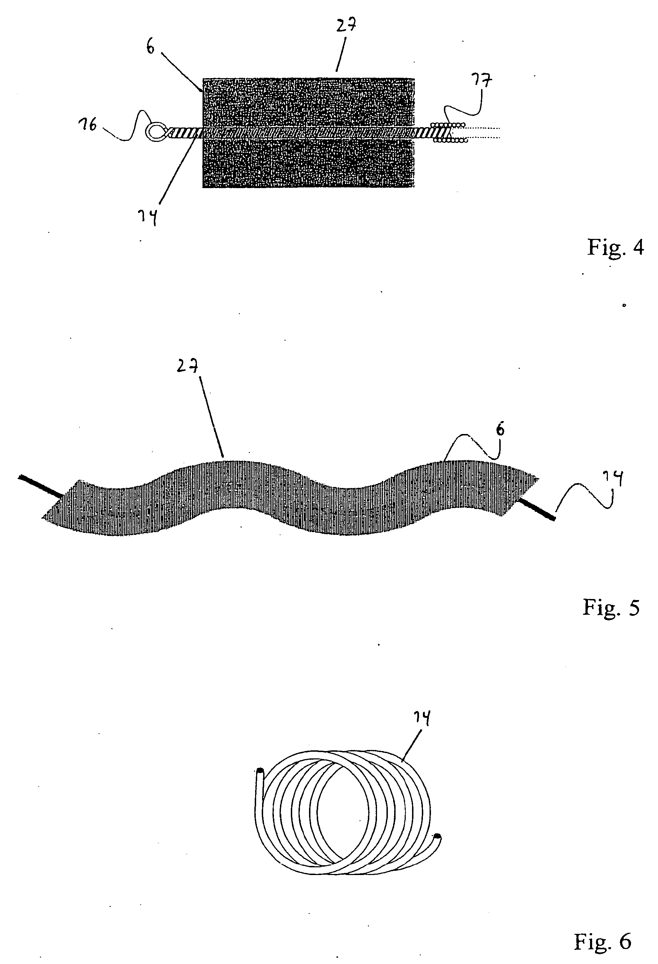

In der Figur 4 ist eine weitere Ausführungsform des erfindungsgemäßen distalen Elements 27 dargestellt, wobei hier die Fasern 6 als einheitliche Fläche dargestellt werden, wie sie sich aufgrund der Dichte des Faserbesatzes ergibt. In diesem Fall sind die Seelendrähte 14 am distalen Ende des distalen Elements über eine Schlaufe 16 miteinander verbunden, so dass letztlich ein einziger Seelendraht 14 vorliegt, der von proximal nach distal verläuft, eine Schlaufe 16 ausbildet und wieder zurück nach proximal verläuft. Es erfolgt somit eine Verdrillung der beiden Enden des Seelendrahts 14.

Des Weiteren ist aus der Figur 4 zu erkennen, wie das distale Element 27 über eine Wendel 17 mit weiter proximal gelegenen Komponenten der Vorrichtung, insbesondere mit einem Führungsdraht 28 verbunden sein kann.

In Figur 5 ist ein wellen- oder sinusförmiger Verlauf des Seelendrahtes 14 dargestellt. Diesem wellenförmigen Verlauf folgen auch die Fasern 6, so dass sich ein wellenförmiger Verlauf des gesamten distalen Elements 27 ergibt. Im Vergleich zu geraden Seelendrähten 14 ist hiermit eine verbesserte Reinigungswirkung verbunden.

In Figur 6 ist eine helixförmige Sekundärstruktur der Seele 14 zu erkennen, wobei hier zur Vereinfachung der Darstellung die Faserstruktur nicht eingezeichnet ist. Zu beachten ist des weiteren, dass die Seele 14 auch hier wiederum aus mindestens zwei miteinander verdrillten Seelendrähten 14 besteht, welche eine primäre Helix der umeinander verwundenen Seelendrähte 14 ausbilden. Die in Figur 6 dargestellte Sekundärhelix darf daher nicht mit der sich durch die Verdrillung der Seelendrähte 14 ergebenden Primärhelix verwechselt werden. Eine helixförmige Struktur des distalen Elements ist zum einen aufgrund der besseren Reinigungswirkung an den Gefäßinnenwänden von Vorteil, zum anderen kann eine solche Ausführungsform dazu dienen, kürzere Bürsten in einem Aneurysma zu platzieren, um dieses zu verschließen.

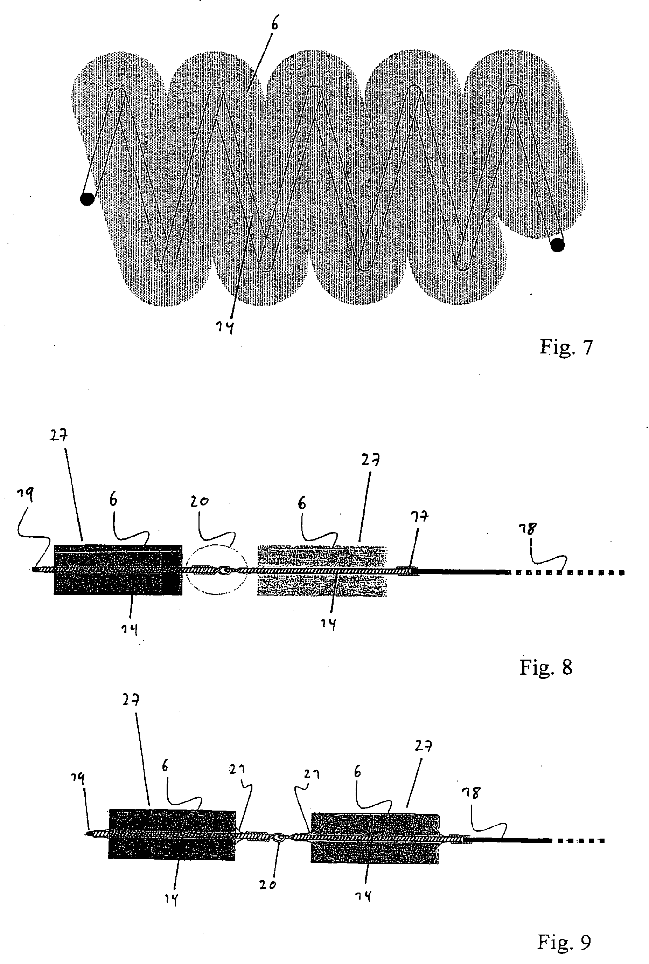

Eine weitere Darstellung eines helixförmigen distalen Elements ist in Figur 7 ersichtlich, wobei hier ein Längsschnitt dargestellt ist, bei dem schematisch der Faserbesatz 6 zu erkennen ist.

Figur 8 zeigt eine Ausführungsform der Erfindung bestehend aus mehreren distalen Elementen 27. Jedes distale Element 27 setzt sich aus miteinander verdrillten Seelendrähten 14 und einem Faserbesatz 6 zusammen. Dabei sind die distalen Elemente 27 miteinander über ein Gelenk 20 verbunden, so dass eine gewisse Flexibilität beim Vor- und Zurückschieben innerhalb der Gefäße gegeben ist. Am proximalen Ende ist das distale Element über eine Wendel 17 mit einem Schub- oder Führungsdraht 18 verbunden. Am distalen Ende weist die Vorrichtung eine abgerundete Spitze 19 auf. Da in diesem Beispiel die Fasern 6 der beiden distalen Elemente 27 verschieden ausgebildet sind, ist die Farbschattierung hier unterschiedlich gewählt.

In Figur 9 ist eine weitere Ausführungsform mit mehreren distalen Elementen dargestellt, die weitgehend der Ausführungsform gemäß Figur 8 entspricht. Zusätzlich umfaßt die Vorrichtung jedoch Streben 21 die vom distalen Ende des distalen Elements 27 ausgehen, radial nach außen hervortreten und am proximalen Ende des distalen Elements 27 wieder zentral zusammen laufen. Die radiale Ausdehnung der Streben 21 entspricht dabei der radialen Ausdehnung der Fasern 6. Eine solche Ausführungsform mit zusätzlichen Streben 21 dient der Verbesserung der Führung des Systems im Gefäß.

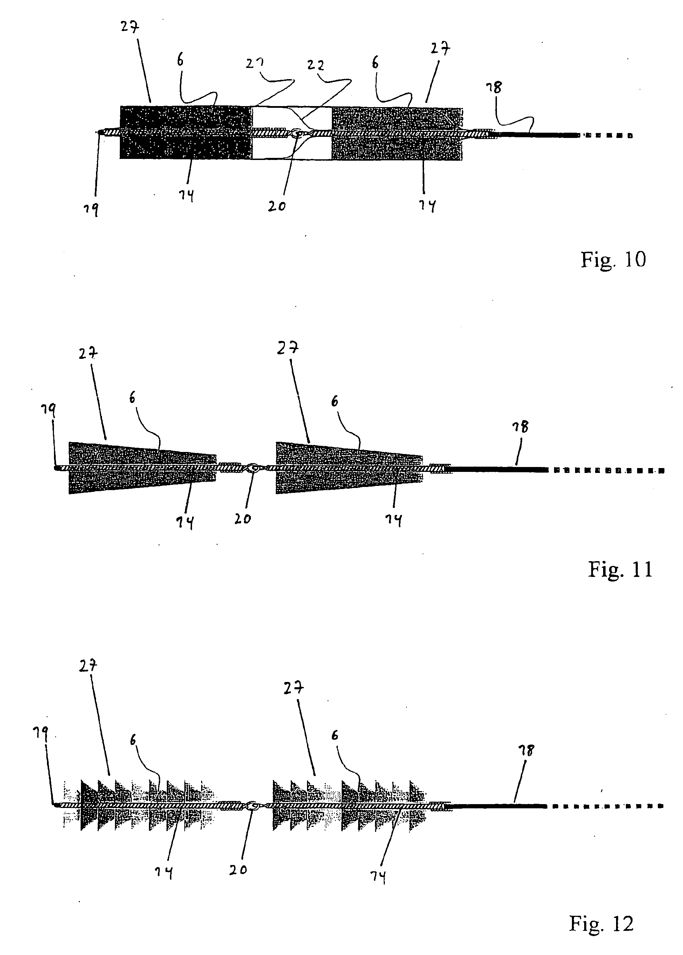

Eine weitere Ausführungsform mit Streben ist in der Figur 10 zu erkennen, wobei sich hier die Streben 21 über sämtliche distalen Elemente 27 erstrecken. Zur zusätzlichen Stabilisierung sind allerdings Zwischenstreben 22 vorgesehen.

In Figur 11 ist eine Ausführungsform mit zwei distalen Elementen zu erkennen, wobei jedes distale Element 27 eine konische Struktur hat. Dabei nimmt die radiale Ausdehnung der Fasern der distalen Elemente 27 von proximal nach distal zu, d. h. die distalen Elemente 27 sind jeweils im distalen Bereich breiter als im proximalen Bereich. Eine solche Ausführungsform hat den Vorteil, dass unabhängig von der Weite der Blutgefäße stets Fasern 6 vorhanden sind, die eine optimale Länge aufweisen.

In Figur 12 ist eine weitere Ausführungsform zu erkennen, bei der jeweils innerhalb eines distalen Elements 27 mehrfach die radiale Ausdehnung der Fasern von proximal nach distal zunimmt. Entsprechend besteht jedes distale Element 27 aus mehreren konusförmigen Bürsten.

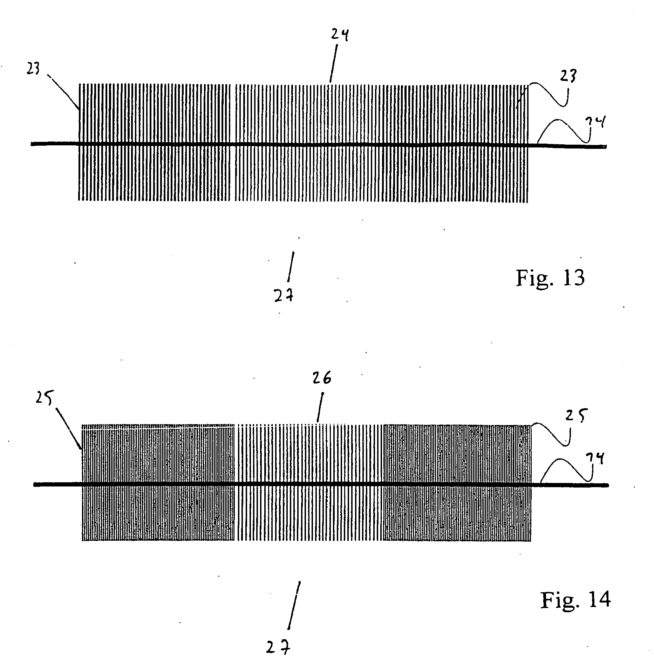

In Figur 13 ist eine Ausführungsform zu erkennen, bei der der mittlere Bereich 27 des distalen Elements 27 weichere Fasern, der proximale sowie der distale Bereich 23 hingegen härtere Fasern aufzeigt. In diesem Fall dient der mittlere Bereich 24 in erster Linie der Aufnahme eine Clots, während die proximalen und distalen Bereiche 23 der Verstärkung der Reinigungswirkung dienen.

Ähnlich verhält es sich bei der in Figur 14 dargestellten Ausführungsform, wobei hier der gleiche Effekt dadurch erzielt wird, dass der Faserbesatz im Bereich 26 dünner ist als in den proximalen und distalen Bereichen 25.

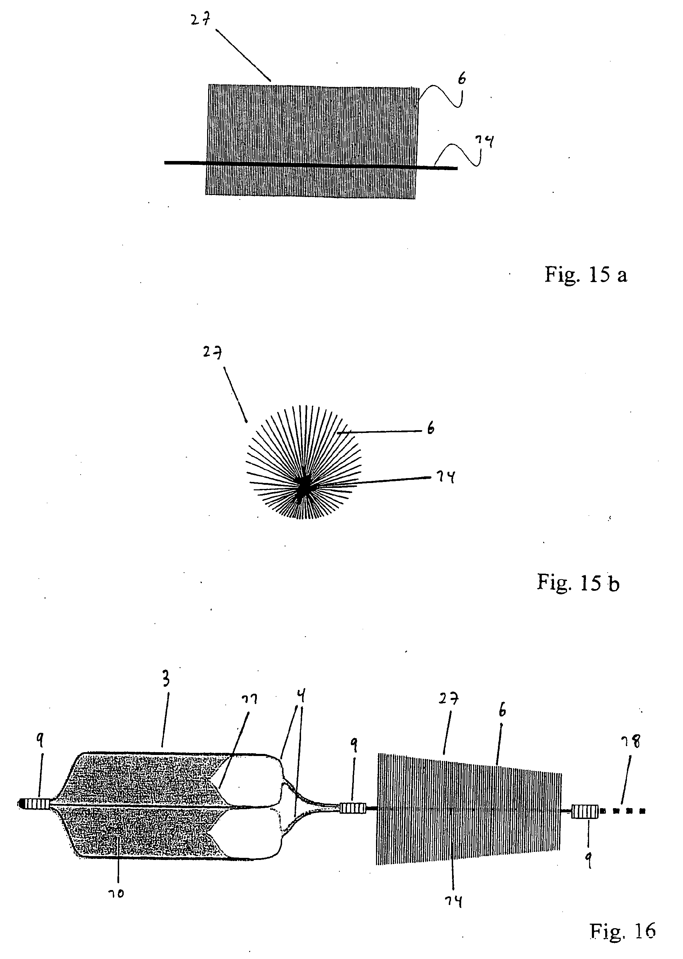

In den Figuren 15a und 15b ist ein distales Element 27 im Längsschnitt sowie in einer proximalen/distalen Ansicht zu erkennen, bei dem die miteinander verdrillten Seelendrähte 14 exzentrisch verlaufen. Entsprechend stehen die Fasern 6 zu einer Seite erheblich weiter hervor als zur anderen Seite. Dies ist insbesondere dann von Vorteil, wenn das distale Element 27 seitlich an einem Thrombus vorbei geführt werden soll.

In Figur 16 ist eine Ausführungsform dargestellt, bei der eine Korbstruktur 3 mit einer Bürstenstruktur kombiniert wurde. Das distale Element 27 besteht dabei wiederum aus miteinander verdrillten Seelendrähten 14 und radial nach außen hervorstehenden Fasern 6 und ist proximal zum Korb 3 angeordnet. Der Korb 3 weist eine Polymerhaut 10 auf, wobei Querstreben 11 die Polymerhaut 10 mit den Streben 4 verbinden. Des Weiteren weist die Vorrichtung röntgendichte Marker 9 und einen Führungsdraht 18 auf. Beim Zurückziehen der Vorrichtung in Richtung proximal wird ein Thrombus zunächst von dem distalen Element 27 mit den Fasern 6 erfasst und festgehalten. Sollten dabei einzelne Thrombusfragmente abgespalten werden, werden diese durch die distal befindliche Korbstruktur 3 mit ihrer Polymerhaut 10 eingefangen, da die Polymerhaut 10 letztlich eine Tasche ausbildet. Durch die Kombination von Bürstenstruktur und Korbstruktur wird eine besonders vorteilhafte Vorrichtung zur Entfernung von Thromben geschaffen.

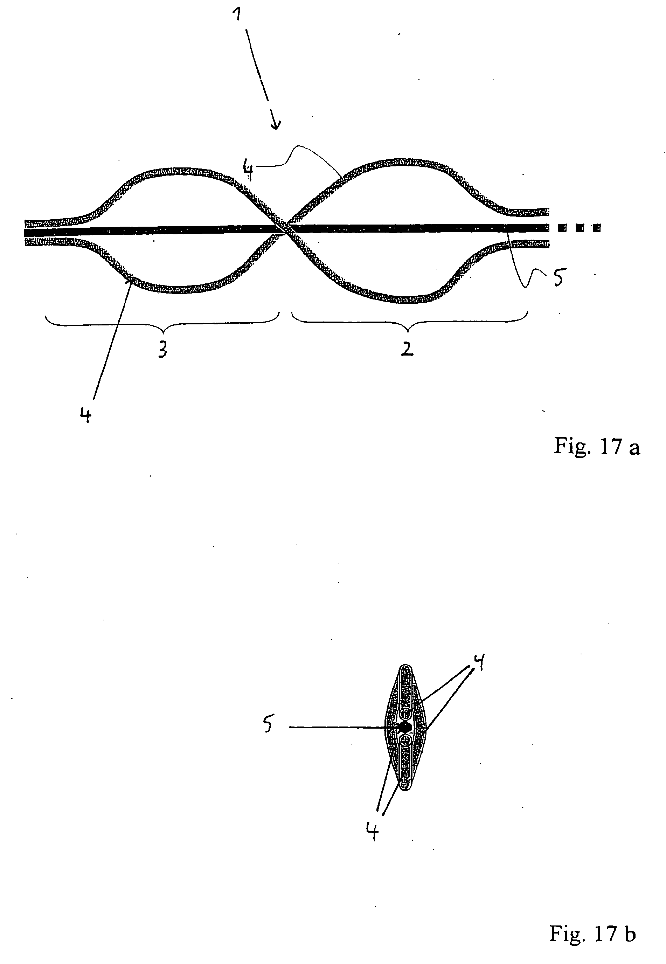

In Figur 17a ist gemäß einer alternativen Ausführungsform der Erfindung eine Korbstruktur 1 bestehend aus einem proximalen Korb 2 und einem distalen Korb 3 gemäß der Erfindung dargestellt. Die beiden einzelnen Körbe 2, 3 werden hier dadurch gebildet, dass die die Körbe 2, 3 ausbildenden Streben 4 sich in der Mitte überkreuzen, wobei ein Versatz der Streben vom proximalen Korb 2 zum distalen Korb 3 um jeweils 180° stattfindet. Zentral durch die Korbstruktur 1 verläuft ein Kernfilament 5, dass am distalen Ende mit den Streben 4 verbunden ist, während die Streben 4 am proximalen Ende gegenüber dem Kernfilament 5 beweglich sind. Auf diese Weise wird eine gewisse Verschiebbarkeit der Korbstruktur 1 in Längsrichtung gewährleistet, die wichtig ist beim Ein- und Ausbringen der Korbstruktur 1 in bzw. aus einem Mikrokatheter.

Dadurch, dass die beiden Körbe 2, 3 durch die gleichen Streben 4 gebildet werden, wird gewährleistet, dass radiale Kräfte von einem Korb zum nächsten übertragen werden und beispielsweise auf den proximalen Korb 2 wirkend Radialkräfte die vom distalen Korb 3 ausgehenden, nach außen wirkenden Radialkräfte erhöhen. Beispielsweise kann zur Bergung besonders fester Thromben lediglich der distale Korb 3 aus einem Mikrokatheter hinausgeschoben werden, während der proximale Korb 2 im Mikrokatheter verbleibt, so dass durch den äußeren Zwang, der durch den Mikrokatheter auf den proximalen Korb 2 ausgeübt wird, die nach außen wirkende Radialkraft des distalen Korbs 3 stark erhöht wird.

Ergänzend sei noch darauf hingewiesen, dass es sich bei der Figur 17a lediglich um eine Prinzipdarstellung handelt, bei der lediglich zwei Streben 4 dargestellt sind. Üblicherweise werden jedoch zur Ausbildung der Korbstruktur 1 mehr Streben 4, beispielsweise drei bis sechs Streben 4 verwendet.

Figur 17b zeigt die Korbstruktur 1 aus Figur 17a in einer Ansicht von distal. In der Mitte verläuft das Kernfilament 5, während die Streben 4 sich in Längsrichtung gesehen einer rechtsdrehenden Schraube entsprechend um das die Längsachse bildende Kernfilament 5 herumwinden.

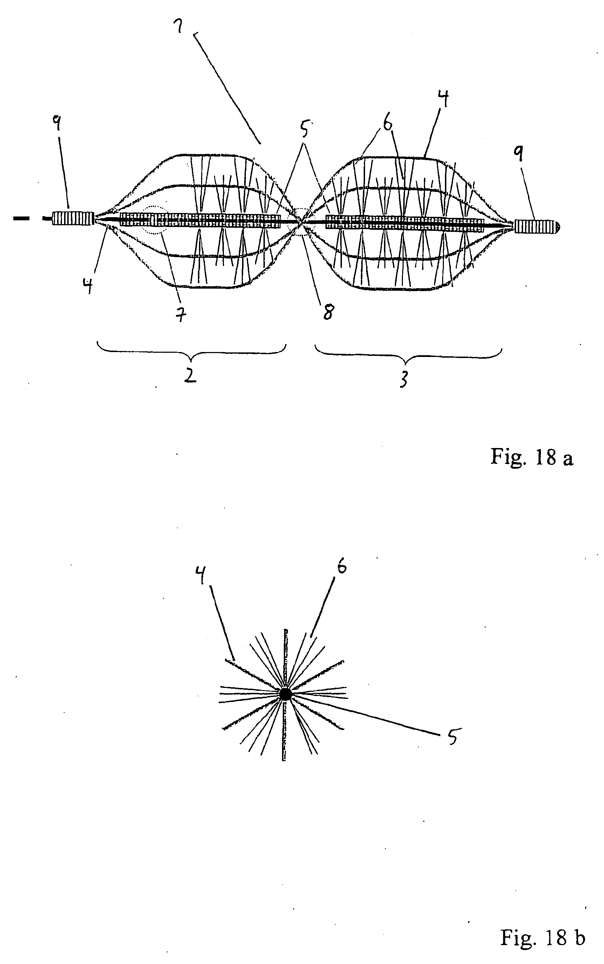

In Figur 18a ist eine weitere Korbstruktur 1 in einer Seitenansicht dargestellt, wobei hier links der proximale Korb 2 und recht der distale Korb 3 zu erkennen ist. Dargestellt sind hier jeweils vier Streben 4, die wiederum beide Körbe 2, 3 ausbilden und sich in einem Knotenpunkt 8 zwischen den beiden Körben 2, 3 schneiden. Durch die Korbstruktur 1 hindurch verläuft wiederum ein Kernfilament 5, auf dem sich jeweils in Längsrichtung verschiebbare Filamente 7 befinden, welche eine Bewehrung aus radial nach außen stehenden Fasern 6 aufweisen. Diese Fasern 6 sind so flexibel, dass sie sich beim Vorschieben der Vorrichtung durch einen Mikrokatheter in Richtung der Längsachse anlegen, nach Ausbringen der Vorrichtung aus dem Mikrokatheter jedoch aufstellen. Die Fasern 6 dienen dazu, einen eingefangenen Thrombus zusätzlich festzuhalten, und bewirken darüber hinaus eine Stabilisierung, die auf die thrombogene Beschichtung der Fasern 6 zurückzuführen ist. Sowohl am distalen Ende als auch am proximalen Ende der Korbstruktur 1 befinden sich jeweils röntgendichte Marker 9, die der Visualisierung des Systems mittels bildgebender Verfahren dienen. Das Kernfilament 5 ist an der mit einem Kreis gekennzeichneten Stelle unterbrochen, so dass beim Zusammenziehen bzw. Auseinanderfalten der Korbstruktur 1 der proximale Teil des Kernfilamentes 5 längsbeweglich im Filament 7 hin- und hergleiten kann. Auf diese Weise wird die Längsdehnung und -kontraktion der Korbstruktur 1 deutlich vereinfacht. Der röntgendichte Marker 9 am distalen Ende der Vorrichtung weist eine abgerundete Spitze auf, die atraumatisch wirkt.

In Figur 18b ist die Korbstruktur 1 aus Figur 18a in einer Ansicht von distal dargestellt, bei der man sechs gleichmäßig über den Umfang verteilte Streben 4 erkennt. Darüber hinaus stehen Fasern 6 bündelweise radial nach außen hervor. Die Längsachse wird wiederum durch das Kernfilament 5 gebildet.

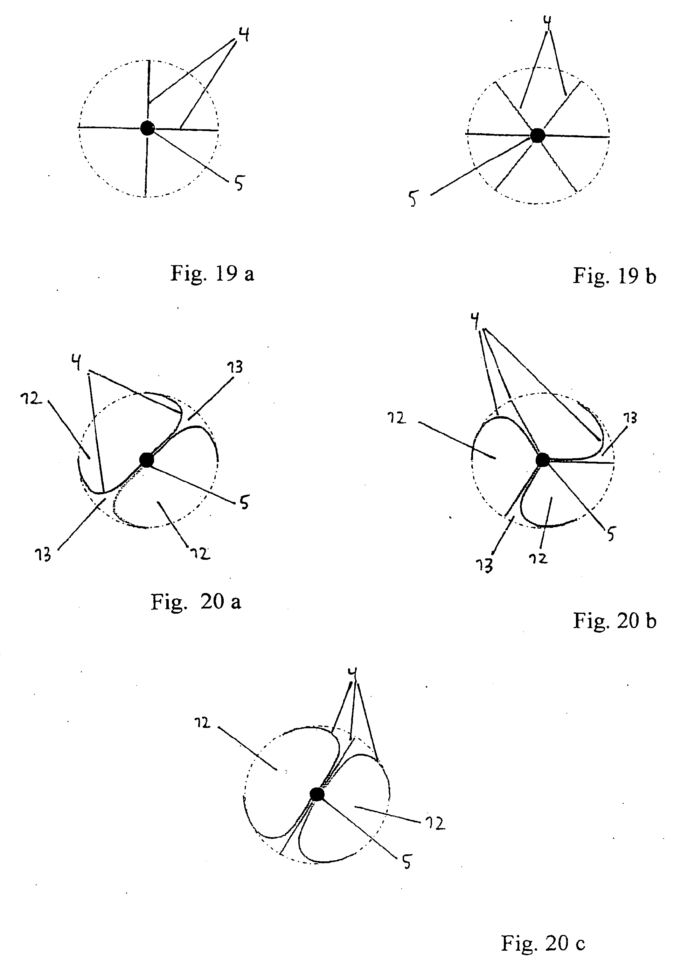

In den Figuren 19a und 19b sind Ansichten proximaler Öffnungen einer Korbstruktur zu erkennen, wobei die Korbstruktur in Figur 19a sich aus vier, die Korbstruktur in Figur 19b aus sechs Streben 4 zusammensetzt. Einerseits bewirkt die Verwendung von sechs Streben 4 eine geschlossenere Korbstruktur 1, andererseits sind jedoch die proximalen Öffnungen der Korbstruktur 1 zwischen den Streben 4 bei Verwendung von lediglich vier Streben 4 deutlich größer. Letzteres kann von Vorteil sein, wenn der Thrombus besonders fest und zusammenhängend ist, da es auf diese Weise einfacher wird, den Thrombus durch die proximale Öffnung hindurch in die Korbstruktur 1 hineinzubefördern.

In den Figuren 20a, 20b und 20c wird eine alternative Möglichkeit gezeigt, die zwischen den Streben 4 befindlichen Teilöffnungen 12, 13 zu vergrößern. Hierbei gehen die Streben 4 am proximalen Ende der Korbstruktur 1 von einem gemeinsamen Punkt aus und verlaufen zunächst gruppiert eng benachbart und parallel in Richtung distal, bevor sie etwas weiter distal eines ersten Abschnitts auseinander laufen, um schließlich ihre Endposition einzunehmen, in der die Streben 4 gleichmäßig über den Umfang des Korbs verteilt sind. Bei der Verwendung von vier Streben erhält man, wie bereits in Figur 19a vier Teilöffnungen, durch den neuartigen Verlauf der Streben werden jedoch die großen Teilöffnungen 12 stark vergrößert, während die kleinen Teilöffnungen 13 erheblich verkleinert werden. Durch den paarweise parallelen, eng benachbarten Verlauf der Streben 4 in der Anfangsphase werden Teilöffnungen 12, 13 geschaffen, die nahezu den Teilöffnungen entsprechen, wie sie sich bei Verwendung der Hälfte der Streben 4 ergeben würden, d. h. in Figur 20a wird die proximale Öffnung im Wesentlichen halbiert, in Figur 20b bei Verwendung von sechs Streben 4 in etwa gedrittelt. In Figur 20c schließlich verlaufen zunächst jeweils drei Streben 4 gruppiert eng benachbart, so dass trotz Verwendung von insgesamt sechs Streben 4 zwei etwa halbkreisförmige Teilöffnungen 12 entstehen.

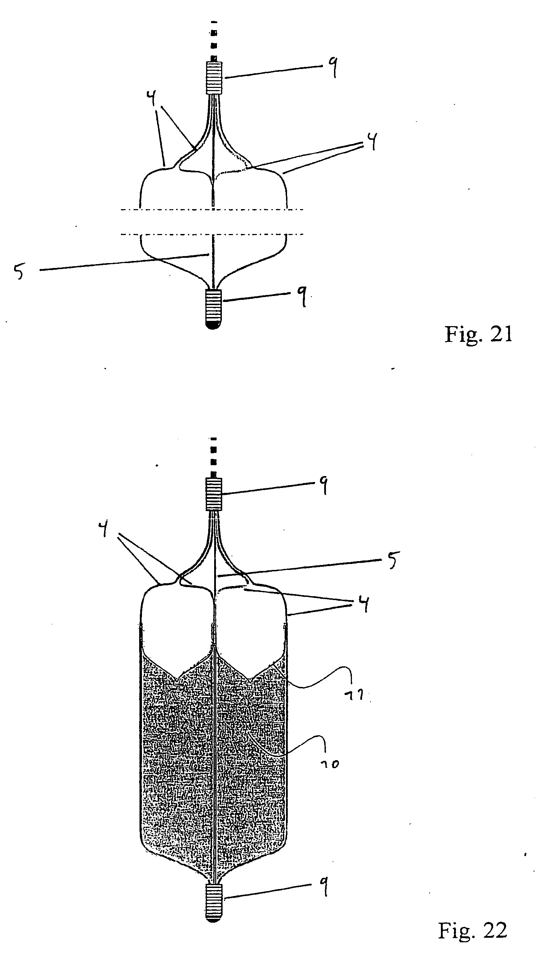

In Figur 21 ist die Korbstruktur 1 aus Figur 20a noch einmal in der Seitenansicht dargestellt, bei der man wiederum erkennt, dass die Streben 4 vom Marker 9 aus zunächst paarweise parallel verlaufen, bevor sie auseinander divergieren, um sich gleichmäßig über den Umfang der Korbstruktur 1 zu verteilen. Es sei angemerkt, dass in Figur 21 lediglich ein Korb der Korbstruktur 1 dargestellt ist und darüber hinaus der Mittelteil ausgelassen wurde.

In Figur 22 schließlich erkennt man eine weitere Korbstruktur 1, wobei hier lediglich der distale Korb 3 zu erkennen ist. Dieser setzt sich wiederum aus vier Streben 4 zusammen, die zwischen zwei röntgendichten Markern 9 verlaufen. Zentral durch die Korbstruktur 1 erstreckt sich das Kernfilament 5. Die Besonderheit der Ausführungsform der Figur 22 liegt in der Polymerhaut 10, die sich am distalen Ende der Korbstruktur 1 befindet. Quer zu den Streben 4 sind dabei auch Querstreben 11 zu erkennen, die jeweils den Abschluss der Polymerhaut 10 nach proximal darstellen. Auf diese Weise wird gewissermaßen am distalen Ende der Korbstruktur 1 eine Tasche gebildet, die der Aufnahme eines Thrombus dient. Die quer verlaufenden Verbindungsstreben 11 dienen der Stabilisierung der Kantenstruktur der Polymerhaut 10 sowie einer zusätzlichen Fixierung der Streben 4 einander gegenüber.

## Patentansprüche

1. Vorrichtung zur Entfernung von Fremdkörpern und Thromben aus Körperhohlräumen und Blutgefäßen mit einem Führungsdraht (18), der ein oder mehrere distale Elemente (27) aufweist, **dadurch gekennzeichnet, dass** das distale Element (27) mindestens zwei miteinander verdrillte Seelendrähte (14) umfasst, zwischen denen Fasern (6) quer zum Verlauf der Seelendrähte (14) eingelegt und mit den Seelendrähten verdrillt sind, so dass die Fasern (6) vom distalen Element (27) radial nach außen hervorstehen, wobei die radiale Ausdehnung der Fasern (6) von proximal nach distal zunimmt.

2. Vorrichtung nach Anspruch 1, **dadurch gekennzeichnet, dass** die verdrillten Seelendrähte (14) geradlinig verlaufen.

3. Vorrichtung nach einem der Ansprüche 1 oder 2, **dadurch gekennzeichnet, dass** die Fasern (6) im proximalen Bereich des distalen Elements (27) härter sind als im distalen Bereich des distalen Elements (27).

4. Vorrichtung nach einem der Ansprüche 1 bis 3, **dadurch gekennzeichnet, dass** die Fasern (6) im mittleren Bereich des distalen Elements (27) weicher sind als im proximalen und im distalen Bereich des distalen Elements (27).

5. Vorrichtung nach einem der Ansprüche 1 bis 4, **dadurch gekennzeichnet, dass** die Dichte des Faserbesatzes im mittleren Bereich des distalen Elements (27) geringer ist als im proximalen und distalen Bereich des distalen Elements (27).

6. Vorrichtung nach einem der Ansprüche 1 bis 5, **dadurch gekennzeichnet, dass** die radial außen liegenden Enden der Fasern (6) Verdickungen aufweisen und/oder zumindest teilweise über Schlaufen miteinander verbunden sind.

7. Vorrichtung nach einem der Ansprüche 1 bis 6, **dadurch gekennzeichnet, dass** die Fasern (6) zumindest teilweise unterschiedlich weit zu beiden Seiten des distalen Elements (27) radial nach außen hervorstehen.

8. Vorrichtung nach einem der Ansprüche 1 bis 7, **dadurch gekennzeichnet, dass** die Seelendrähte (14) im Querschnitt des distalen Elements (27) außerhalb des Zentrums verlaufen.

9. Vorrichtung nach einem der Ansprüche 1 bis 8, **dadurch gekennzeichnet, dass** die durch das oder die distalen Elemente ausgebildete Bürstenstruktur geeignet ist, sich unter dem äußeren Zwang eines Mikrokatheters eng zusammenzufalten, um innerhalb des Mikrokatheters transportiert zu werden und sich bei Wegfall des äußeren Zwangs durch den Mikrokatheter zur vollen Bürstenstruktur entfaltet.

10. Vorrichtung nach einem der Ansprüche 1 bis 9, **dadurch gekennzeichnet, dass** die Fasern (6) einen Winkel von 70° bis 110°, vorzugsweise einen Winkel von 80° bis 90°, zur Längsachse der Vorrichtung ausbilden.

11. Vorrichtung nach einem der Ansprüche 1 bis 10, **dadurch gekennzeichnet, dass** die Fasern (6) mit einer Beschichtung versehen sind.

12. Vorrichtung nach einem der Ansprüche 1 bis 11, **gekennzeichnet durch** einen oder mehre röntgendichte Marker (9).

13. Vorrichtung nach einem der vorstehenden Ansprüche, **dadurch gekennzeichnet, dass** die Seelendrähte (14) aus Platin oder einer Platinlegierung, insbesondere aus Platin-Iridium, bestehen, wobei die Fasern (6) durch Einklemmen, Kleben, Verknoten und/oder Anschmelzen an den Seelendrähten (14) festgelegt sind.

14. Vorrichtung nach einem der Ansprüche 1 bis 13 in Kombination mit einem Führungskatheter und/oder Mikrokatheter, wobei der Führungs- oder Mikrokatheter ein Aspirationskatheter sein kann.

15. Vorrichtung zur Entfernung von Fremdkörpern und Thromben aus Körperhohlräumen und Blutgefäßen nach einem der Ansprüche 1 bis 14, mit einem Führungsdraht (18), der ein oder mehrere distale Elemente (27) aufweist, wobei das distale Element (27) radial nach außen hervorstehende Fasern (6) aufweist, **dadurch gekennzeichnet, dass** das distale Element (27) eine Konusform aufweist.

## Claims

1. Device for the removal of foreign objects and thrombi from body cavities and blood vessels comprising a guide wire (18) provided with one or several distal elements (27), **characterized in that** the distal element (27) comprises at least two core wires (14) which are twisted around each other and between which fibers (6) are arranged transversely to the extension of the core wires (14), with said fibers (6) being twisted together with the core wires (14) so that the fibers (6) project radially outward from the distal element (27) and wherein the radial extension of the fibers (6) of the distal element (27) increases from proximal to distal.

2. Device according to claim 1, **characterized in that** the twisted core wires (14) extend in a straight line.

3. Device according to claim 1 or 2, **characterized in that** the fibers (6) in the proximal area of the distal element (27) are harder than in the distal area of the distal element (27).

4. Device according to any one of claims 1 to 3. **characterized in that** the fibers (6) in the middle area of the distal element (27) are softer than in the proximal and distal area of the distal element (27).

5. Device according to any one of claims 1 to 4, **characterized in that** the density of the fiber coverage in the middle area of the distal element (27) is lower than in the proximal and distal area of the distal element (27).

6. Device according to any one of claims 1 to 5, **characterized in that** the ends of the fibers (6) located radially outward are provided with slubs and/or are at least in part connected with each other by means of loops.

7. Device according to any one of claims 1 to 6, **characterized in that** the fibers (6), at least partially, protrude differently far radially outward at both sides of the distal element (27).

8. Device according to any one of claims 1 to 7, **characterized in that** the core wires (14) in the cross sectional area of the distal element (27) are arranged eccentrically.

9. Device according to any one of claims 1 to 8, **characterized in that** the brush structure formed by one or several distal elements is suitable to be flatly collapsible under the external strain exerted by a micro-catheter to be transported inside the micro-catheter and unfolds to its full brush structure when said external strain caused by the micro-catheter is omitted.

10. Device according to any one of claims 1 to 9, **characterized in that** the fibers (6) form an angle with the longitudinal axis of the device that ranges between 70° and 110°, preferably between 80° and 90°.

11. Device according to any one of claims 1 to 10, **characterized in that** the fibers (6) are provided with a coating.

12. Device according to any one of claims 1 to 11, **characterized by** one or several radiopaque markers (9).

13. Device according to any one of the preceding claims, **characterized in that** the core wires (14) are made of platinum or a platinum alloy, preferably platinum-iridium, wherein the fibers (6) are secured or attached to the core wires (14) by clamping, bonding, knotting and/or fusing.

14. Device according to any one of claims 1 to 13 in combination with a guide catheter and/or micro-catheter, wherein the guide or micro-catheter can be designed as an aspiration catheter.

15. Device for the removal of foreign objects and thrombi from body cavities and blood vessels according to any of claims 1 to 14 comprising a guide wire (18) provided with one or several distal elements (27), with the distal element (27) being provided with fibers (6) projecting radially outward, **characterized in that** the distal element (27) has a tapered shape.

## Revendications

1. Dispositif pour éliminer des corps étrangers et des tromboses d'espaces creux du corps et de vaisseaux sanguins avec un fil de guidage (18) qui présente un ou plusieurs éléments distaux (27), **caractérisé en ce que** l'élément distal (27) comprend au moins deux fils d'âme assemblés par torsion, entre lesquels sont placées des fibres (6) transversalement à l'extension des fils d'âme (14) et sont assemblées par torsion avec les fils d'âme de sorte que les fibres (6) font saillie de l'élément distal (27) radialement vers l'extérieur, où l'extension radiale des fibres (6) augmente de proximal à distal.

2. Dispositif selon la revendication 1, **caractérisé en ce que** les fils d'âme assemblés par torsion (14) s'étendent d'une manière rectiligne.

3. Dispositif selon l'une des revendications 1 ou 2, **caractérisé en ce que** les fibres (6) dans la zone proximale de l'élément distal (27) sont plus dures que dans la zone distale de l'élément distal (27).

4. Dispositif selon l'une des revendications 1 à 3, **caractérisé en ce que** les fibres (6) dans la zone médiane de l'élément distal (27) sont plus molles que dans la sone proximale et distale de l'élément distal (27).

5. Dispositif selon l'une des revendications 1 à 4, **caractérisé en ce que** la densité de la garniture de fibres dans la zone médiane de l'élément distal (27) est plus faible que dans la zone proximale et distale de l'élément distal (27).

6. Dispositif selon l'une des revendications 1 à 5, **caractérisé en ce que** les extrémités des fibres (6) situées radialement à l'extérieur présentent des épaississements et/ou sont reliées au moins partiellement entre elles par des boucles.

7. Dispositif selon l'une des revendications 1 à 6, **caractérisé en ce que** les fibres (6) font saillie radialement vers l'extérieur, au moins partiellement sur les largeurs différentes, des deux côtés de l'élément distal (27).

8. Dispositif selon l'une des revendications 1 à 7, **caractérisé en ce que** les fils d'âme (14), en section transversale de l'élément distal (27), s'étendent à l'extérieur du centre.

9. Dispositif selon l'une des revendications 1 à 8, **caractérisé en ce que** la structure de brosse formée par le ou les éléments distaux convient pour se replier étroitement sous la contrainte extérieure d'un micro-cathéter, pour être transportée à l'intérieur du micro-cathéter et pour se déployer, lors de la suppression de la contrainte extérieure à travers le micro-cathéter en structure de brosse complète.

10. Dispositif selon l'une des revendications 1 à 9, **caractérisé en ce que** les fibres (6) forment un angle de 70° à 110°, de préférence un angle de 80° à 90° relativement à l'axe longitudinal du dispositif.

11. Dispositif selon l'une des revendications 1 à 10, **caractérisé en ce que** les fibres (6) sont pourvues d'un revêtement.

12. Dispositif selon l'une des revendications 1 à 11, **caractérisé par** un ou plusieurs marqueurs anti-radiations (9)

13. Dispositif selon l'une des revendications précédentes, **caractérisé en ce que** les fils d'âme (14) sont réalisés en platine ou en un alliage de platine, en particulier en platine-iridium, où les fibres (6) sont fixées par serrage, collage, nouage et/ou fusion aux fils d'âme (14).

14. Dispositif selon l'une des revendications 1 à 13, en combinaison avec un cathéter de guidage et/ou un micro-cathéter, ou le cathéter de guidage ou micro-cathéter peut être un cathéter d'aspiration.

15. Dispositif pour éliminer des corps étrangers et des thromboses d'espaces du corps et de vaisseaux sanguins selon l'une des revendications 1 à 14, avec un fil de guidage (18) qui présente un ou plusieurs éléments distaux (27), où l'élément distal (27) présente des fibres (6) faisant saillie radialement vers l'extérieur, **caractérisé en ce que** l'élément distal (27) présente une forme conique.
